(19) Europäisches Patentamt
European Patent Office
Office européen des brevets

(11) EP 4 082 549 A1

(12) **EUROPEAN PATENT APPLICATION**
published in accordance with Art. 153(4) EPC

(43) Date of publication:
**02.11.2022 Bulletin 2022/44**

(21) Application number: **20905856.9**

(22) Date of filing: **23.12.2020**

(51) International Patent Classification (IPC):
***A61K 31/5585*** *(2006.01)* ***A61K 9/06*** *(2006.01)*
***A61K 9/20*** *(2006.01)* ***A61K 45/00*** *(2006.01)*
***A61K 47/38*** *(2006.01)* ***A61P 13/12*** *(2006.01)*
***A61P 43/00*** *(2006.01)*

(52) Cooperative Patent Classification (CPC):
**A61K 9/06; A61K 9/20; A61K 31/5585; A61K 45/00; A61K 47/38; A61P 13/12; A61P 43/00**

(86) International application number:
**PCT/JP2020/048091**

(87) International publication number:
**WO 2021/132302 (01.07.2021 Gazette 2021/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.12.2019 JP 2019231495**

(71) Applicant: **Toray Industries, Inc.**
**Tokyo, 103-8666 (JP)**

(72) Inventors:
- **KURUMATANI Hajimu**
  **Tokyo 103-8666 (JP)**
- **OKADA Kiyonobu**
  **Tokyo 103-8666 (JP)**
- **YAMADA Naohiro**
  **Tokyo 103-8666 (JP)**
- **KIRIYAMA Takashi**
  **Tokyo 103-8411 (JP)**
- **KANO Hiroyuki**
  **Tokyo 103-8411 (JP)**
- **YAMADA Shunsuke**
  **Tokyo 103-8411 (JP)**

(74) Representative: **Kador & Partner PartG mbB**
**Corneliusstraße 15**
**80469 München (DE)**

# (54) DRUG FOR PREVENTING DIALYSIS SHIFT OR RENAL DEATH

(57) Provided is a drug for preventing dialysis shift or renal death, which can prevent dialysis shift or renal death by administering, to a specific patient group, a sustained-release preparation comprising a compound represented by the general formula (I) as an active ingredient. Provided is a drug for preventing dialysis shift or renal death, which has an oral sustained-release preparation comprising a compound represented by the following general formula (I), wherein R represents hydrogen or a pharmacologically acceptable cation, as an active ingredient, wherein the drug is used in such a manner that the compound represented by the general formula (I) is administered at 220 to 260 μg per day to a primary glomerular disease or nephrosclerosis patient with a serum creatinine level of 2.0 mg/dl or more and less than 3.0 mg/dl, and/or with a nutritional disorder.

Fig. 1

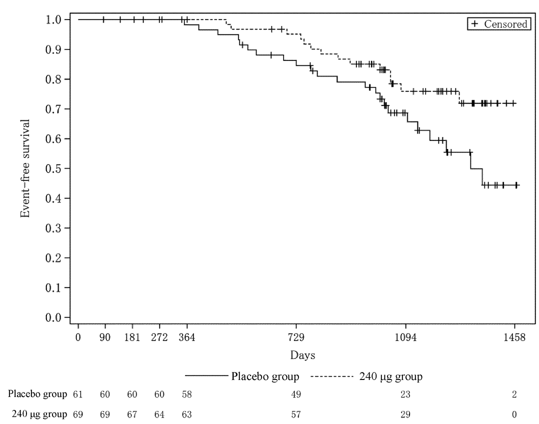


EP 4 082 549 A1

## Description

Technical Field

The present invention relates to a drug for preventing dialysis shift or renal death to be administered to specific patient groups.

Background Art

The number of patients with end-stage renal failure requiring dialysis or renal transplantation continues to increase worldwide, and its significant social burden has become a problem.

Primary diseases that lead to end-stage renal failure include primary glomerular diseases such as chronic glomerulonephritis, secondary glomerular diseases such as diabetic nephropathy, tubulointerstitial nephritis, and the like. Among chronic renal failures, those with diabetic nephropathy as a primary disease and those with non-diabetic nephropathy as a primary disease have different pathological conditions. In the severity classification of chronic kidney disease (hereinafter abbreviated as CKD) revised in 2012 by Kidney Disease: Improving Global Outcomes (hereinafter abbreviated as KDIGO), which is an international kidney disease guideline, chronic renal failures have been roughly divided into those with diabetes as a primary disease and those with non-diabetes as a primary disease.

Primary glomerular diseases and nephrosclerosis are major parts of non-diabetic renal damage or chronic renal failure. In recent years, with the increase in diabetes, the proportion of diabetic nephropathy in the causative disease of dialysis is increasing all over the world. On the other hand, in East Asia, primary glomerular diseases such as IgA nephropathy occupy the top or second reason for dialysis, and particularly in China, they occupy the top cause of dialysis. Further, nephrosclerosis is also the third leading reason for dialysis in most Asian countries, and tends to continue to increase worldwide with the increase in arteriosclerosis in recent years.

Further, new diabetic therapeutic agents have recently become widely used for the treatment of diabetes, and the patient's glycemic control is now much better. Accordingly, with treatment with these drugs, the prognosis of diabetic nephropathy is expected to improve, and some drugs have obtained good results in large-scale clinical trials. However, currently, methods for treating non-diabetic chronic renal damage are not very advanced.

Since damaged glomeruli are not regenerated, the purpose of treatment of chronic renal damage is to make the progression of renal damage as slow as possible. Current actual treatments include salt restriction and diet therapy centered on a low-protein diet, as well as use of antihypertensive drugs including angiotensin-converting enzyme inhibitors (hereinafter, abbreviated as ACEIs) and angiotensin receptor blockers (hereinafter, abbreviated as ARBs). If dialysis is approaching and uremic symptoms are observed, spherical adsorptive carbon (sold in Japan, South Korea, and Taiwan) is used to adsorb and remove causative substances from the digestive tract.

However, even with these treatments, the number of patients with end-stage renal failure continues to increase worldwide, and there is a longing for new therapies that can reliably slow the progression of chronic renal failure.

Beraprost sodium (hereinafter, abbreviated as BPS) is a prostacyclin (also referred to as PGI2) derivative invented in Japan, and is widely used as a therapeutic agent for chronic arterial occlusion and pulmonary arterial hypertension in various Asian countries including Japan. A chronic renal failure prophylactic or therapeutic agent comprising BPS as an active ingredient has been reported (Patent Literature 1). Patent Literature 1 indicates that administration of BPS to renal failure model rats suppresses the increase in serum creatinine levels and reduces renal tissue damage. Moreover, it has been reported that administration of BPS to cats with spontaneous chronic kidney disease suppresses the decline in renal function (Non-Patent Literature 1).

It has also been reported that when BPS is administered to patients with chronic renal failure at 20 μg per dose three times a day for 6 months, the slope of the time-varying straight line of the reciprocal of the serum creatinine level, which indicates the rate of decline in renal function, becomes gentle in the BPS administration group (Non-Patent Literature 2). The drugs used in this clinical case are BPS immediate-release tablets, Dorner (trademark) Tablets (Astellas Pharma Inc.) or Procylin (trademark) Tablets (Kaken Pharmaceutical Co., Ltd.), which were approved for manufacturing and marketing as pharmaceutical products in Japan at that time.

Furthermore, following Non-Patent Literature 2, the results of long-term continuous administration of BPS at 20 μg per dose three times a day for up to 54 months to patients with chronic renal failure have been reported (Non-Patent Literature 3). The results have reported that a patient group with a serum creatinine level of 1.9 mg/dl or less before the start of BPS administration did not shift to dialysis for up to 54 months, whereas a patient group with a serum creatinine level of 2.8 mg/dl or more before the start of BPS administration shifted to dialysis within 24 months, and that in a patient group with a serum creatinine level of 1.9 to 2.8 mg/dl, the effect of BPS was not observed when the serum creatinine level was 2.2 mg/dl or more.

Since it is necessary to take BPS immediate-release tablets three or four times a day, BPS sustained-release tablets to be administered twice a day have been developed. Manufacturing and marketing approval was obtained in

Japan for pulmonary arterial hypertension, and they have been clinically applied as Careload (trademark) LA Tablets (Astellas Pharma Inc.) or Berasus (trademark) LA Tablets (Kaken Pharmaceutical Co., Ltd.). A clinical trial was conducted on chronic renal failure patients with primary glomerular disease and nephrosclerosis as primary diseases as subjects using the BPS sustained-release tablets.

First, as a dose-ranging study, a trial was conducted using the BPS sustained-release tablets in which the usage and dosage were a daily dose of 120 μg or 240 μg, and administration twice a day for 6 months. The difference in the slope between the treatment period and the observation period in the time-varying straight line of the reciprocal of creatinine, which is the primary endpoint, was not superior to the placebo group in the 240-μg group. Neither was the superiority of the 120-μg group over the placebo group. Regarding this endpoint, all actual drug groups exceeded the placebo group; however, effectiveness was higher in the 120-μg group, and the dose-response was not clear. As an indicator of effectiveness, the serum creatinine level during the administration period increased 1.169 times in the placebo group compared to before the start of administration, whereas in the 120-μg and 240-μg BPS administration groups, the increase was suppressed by 1.069 and 1.064 times, respectively, suggesting effectiveness (Non-Patent Literature 4).

Based on the above results, a P-IIb/III trial was designed (Non-Patent Literature 5), and conducted in 7 Asian countries including Japan (the CASSIOPEIR trial). This is a trial whose primary endpoint is whether administration of 120 μg or 240 μg BPS sustained-release tablets prolongs the time to onset compared to placebo administration, using doubling of serum creatinine levels, reaching 6 mg/ml, dialysis shift, and renal transplantation as renal composite endpoints. The administration period of BPS was 2 to 4 years.

The target patients were the same chronic renal failure patients with primary glomerular disease and nephrosclerosis as primary diseases as in the Phase-II trial, and the serum creatinine level at the time of entry was 2.0 to 4.5 mg/dl. The results of this trial show that not only the renal composite endpoints, but also the time to shift to dialysis are not minimized at all in the 120 μg BPS administration group or the 240 μg BPS administration group, as reported in Non-Patent Literature 6.

It has been investigated that in humans, how much the estimated glomerular filtration rate (hereinafter abbreviated as eGFR), which is indicated in consideration of serum creatinine levels, gender, and optionally race as an indicator for renal function evaluation, is reduced to be able to predict dialysis shift or renal death. As a result, it has been reported that a 30% to 40% decrease in eGFR (corresponding to a 30% to 40% or higher increase in serum creatinine levels) from the initial value is useful as an indicator to predict the arrival at dialysis or transplantation, whereas a decrease rate less than the above is not appropriate for predicting dialysis or transplantation (Non-Patent Literature 7). In light of the above, the prolongation of time to dialysis or transplantation could not be predicted from the finding that in humans, the serum creatinine level increased 1.167 times in the placebo group compared to before administration, but the increase was suppressed by 1.069 or 1.064 times by BPS administration, which was the result of the Phase-II trial. In fact, no prevention of dialysis or renal death was observed in actual clinical trials with dialysis shit as an event (Non-Patent Literature 6).

In recent years, it has been particularly noted that nutritional disorder, particularly malnutrition, in chronic renal failure patients is an important risk factor not only for the progression of chronic renal failure but also for prognosis. As nutritional disorders in patients with chronic renal failure are qualitatively different from mere malnutrition, the International Society of Renal Disease Nutrition & Metabolism and the International Society of Nephrology advocated in 2008 a new concept of protein energy wasting (hereinafter, abbreviated as PEW). PEW refers to malnutrition caused by a decrease in the storage of body proteins (proteins in skeletal muscle and blood) and energy source (body fat) in renal failure patients. Moreover, PEW becomes a condition that causes a decrease in appetite associated with appetite-related hormonal abnormalities in the gastrointestinal and central nervous system, and that causes sarcopenia (muscle mass loss) in which not only fat but also body proteins such as muscle are lost due to increased protein catabolism and energy metabolism caused by metabolic acidosis, inflammation, etc.

Normal malnutrition results from a relative lack of nutritional intake; however, in patients with renal failure, factors related to uremic symptoms, inflammation, and exhaustion such as increased catabolism of body proteins overlap, so that there may also be a loss of lean body mass that is not associated with a decrease in nutritional intake. This is considered as one of the features of PEW.

PEW can be diagnosed according to the diagnostic criteria shown in Table 1 below (Non-Patent Literature 8). There are four major categories, including "serum chemistry," "body mass," "muscle mass," and "dietary intake." Patients who satisfy 3 or more of the 4 categories, in which at least one item in each category is satisfied, are diagnosed as PEW. It is said that about 30 to 50% of CKD patients during the conservative period meet the diagnostic criteria for PEW.

[Table 1]

| Diagnostic criteria | |
|---|---|
| Serum chemistry | Serum albumin<3.8g/dl<br>Serum prealbumin (transthyretin)<30mg/dl |

(continued)

| Diagnostic criteria | |
|---|---|
| Body mass | Serum cholesterol < 100mg/dl<br>BMI<23<br>Unintended loss of body weight: 5%/3 months or 10%/6 months<br>Body-fat percentage< 10% |
| Muscle mass | Muscle wasting: loss of muscle mass: 5%/3 months or 10%/6 months<br>10% or more decrease in arm muscle circumference |
| Dietary intake | Dietary protein intake < 0.6 g/kg/day continues for at least 2 months<br>Dietary energy intake < 25 kcal/kg/day continues for at least 2 months |

PEW, which is malnutrition of renal disease patients, is of course a poor prognosis factor, and it is very important not to cause PEW. Further, general nutritional status endpoints are also included in the PEW diagnostic criteria. Therefore, even if patients do not meet the PEW diagnostic criteria (even if they do not meet 3 or more of the 4 categories, in which at least one item is satisfied), it is considered important to carry out nutritional management to reduce the number of applicable items as much as possible (Non-Patent Literature 8).

Total lymphocyte count (TLC) is also often used to assess malnutrition and is an indicator of immune capacity. This is represented by TLC (/$mm^3$) = WBC (white blood cell count) x TLC% (percentage of lymphocyte count in white blood cells)/100. A TLC of less than 1,500/$mm^3$ is an indicator of moderate malnutrition.

For chronic renal failure, in order to slow the decline in renal function as much as possible, diet therapy centered on limiting protein intake is often implemented. In addition, the large number of elderly patients at risk of nutritional disorders is also a risk factor for nutritional disorders.

Cachexia is defined as "a syndrome of complex metabolic disorders caused by underlying disease and characterized by a decrease in muscle mass with or without a decrease in fat mass," and can be regarded as an advanced stage of PEW. The clinical feature of cachexia is weight loss in adults and failure to thrive in children.

Further, sarcopenia is defined by "decrease in skeletal muscle mass and decrease in muscle strength or physical function (walking speed, etc.) observed in old age." Following the announcement of operational definitions from the European Working Group on Sarcopenia in Older People (hereinafter, abbreviated as EWGSOP), various definitions such as the Asian Working Group for Sarcopenia (hereinafter abbreviated as AWGS) based on epidemiological data of Asians including Japanese have been reported. All of these require a decrease in skeletal muscle mass and include either or both muscle weakness or loss of physical function (Non-Patent Literature 9). In recent years, decreased skeletal muscle mass due to various pathological conditions, so-called secondary sarcopenia, is attracting attention. Chronic kidney disease or chronic renal failure is also known as a disease that frequently causes secondary sarcopenia. Restriction of protein intake to alleviate the decline in renal function is also considered to be involved in the onset and progression of sarcopenia in chronic kidney disease patients.

Frailty is defined as "a state in which mental and physical vitality (motor function, cognitive function, etc.) declines with aging, there are also effects such as the coexistence of multiple chronic diseases, life function is impaired, and mental and physical fragility appears, whereas a state image that can maintain and improve living functions through appropriate intervention and support" (Non-Patent Literature 10), and means a middle ground between a healthy state and a long-term care state that requires support in daily life. Many definitions have been proposed for frailty so far (Non-Patent Literature 11). When frailty is combined in chronic renal failure patients, the prognosis is found to be poor.

Recently, the results of large-scale clinical trials have been announced that SGLT2 inhibitors are extremely effective for diabetic nephropathy, and this announcement is attracting attention. However, the main action mechanism of SGLT2 inhibitors is to inhibit the reabsorption of sugar from the renal tubules, which results in weight loss. It is known that not only the decrease in body fat but also the decrease in muscle mass are involved in this weight loss to the same extent. Accordingly, administration to patients with nutritional disorders such as PEW, and patients with sarcopenia and frailty who had a low muscle mass was not always easy to use because it promoted these disorders and the risk of worsening renal failure and increased mortality risk could not be ruled out.

Moreover, it has been reported that in chronic renal failure rat models, the combined use of BPS with angiotensin inhibitors, such as ARBs and ACEIs, enhances the effect of the angiotensin inhibitors to suppress the progression of renal damage, that is, the effect of suppressing the increase in serum creatinine levels (Patent Literature 2). In this example, however, it is only described that ARB and ACEI have the same combined effect in renal failure rat models; it is not suggested whether the combined use of BPS and ACEI can prevent dialysis or renal death in clinical practice.

Thus, it has been considered from the conventionally known documents that in either case of BPS immediate-release tablets or sustained-release tablets, the time to dialysis shift and to renal death defined by dialysis or kidney

transplantation is not prolonged in humans.

Citation List

Patent Literature

Patent Literature 1: WO 2000/067748
Patent Literature 2: WO 2004/098611

Non-Patent Literature

Non-Patent Literature 1: Takenaka et al., J Vet Intern Med (2018) 32, 236.
Non-Patent Literature 2: Fujita et al., Prostaglandins, Leukotrienes and Essential Fatty Acids (2001) 65(4), 223-227.
Non-Patent Literature 3: Fujita et al., Vascular Biology & Medicine (2006), Vol. 7, p. 281.
Non-Patent Literature 4: Koyama et al., BMC Nephrology (2015) 16, 165.
Non-Patent Literature 5: Nakamoto et al., BMC Nephrology (2014) 15, 153.
Non-Patent Literature 6: Nakamoto et al., Ther Apher Dial. 2019 May 23. doi: 10.1111/1744-9987.12840.
Non-Patent Literature 7: Levey et al., Am J Kidney Dis. (2014) 64, 821-835.
Non-Patent Literature 8: Hamada et al., Shikoku Acta Medica, Vol. 69, No. 5,6, pp. 211-214, 2013.
Non-Patent Literature 9: Clinical Guidelines for Sarcopenia, 2017, page 2.
Non-Patent Literature 10: 2015 Ministry of Health, Labor and Welfare Research Grant "Study on Health Business for Late-Stage Elderly" 2015 Summary/Shared Research Report
Non-Patent Literature 11: Nofuji et al., Community Medicine, Vol. 32, No. 4, pp. 312-320, 2018.
Non-Patent Literature 12: Kajikawa et al., Arzneimittelforschung (1989) 39, 495-9.
Non-Patent Literature 13: Shimamura et al., J Clin Pharmacol. (2017) 57, 524-535.
Non-Patent Literature 14: Levey, et al., Ann Intern Med 150 (2009), 604-612.
Non-Patent Literature 15: Japanese Society of Nephrology, Evidence-based Practice Guideline for the Treatment of CKD, (2009), page 3

Summary of Invention

Technical Problem

An object of the present invention is to provide a drug that suppresses dialysis shift or renal death by administering a sustained-release preparation comprising a compound represented by the formula (I) as an active ingredient to specific patient groups.

Solution to Problem

Specifically, the summary of the present invention is as described below.

(1) A drug for preventing dialysis shift or renal death, which is a sustained-release preparation comprising, as an active ingredient, a compound represented by the following general formula (I):

[Formula 1]

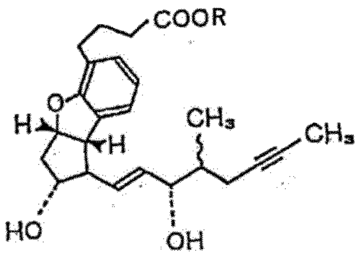

... (I)

wherein R represents hydrogen or a pharmacologically acceptable cation,
wherein the drug is used in such a manner that the compound represented by the general formula (I) is administered at 220 to 260 μg per day to a primary glomerular disease or nephrosclerosis patient with a serum creatinine level of 2.0 mg/dl or more and less than 3.0 mg/dl.
(2) The drug according to the above (1), wherein the compound represented by the general formula (I) is beraprost sodium.
(3) The drug according to the above (1) or (2), wherein the primary glomerular disease or nephrosclerosis patient has an estimated glomerular filtration rate, as calculated by the Chronic Kidney Disease Epidemiology Collaboration (hereinafter, abbreviated as CKD-EPI) equation, of 15 ml/min/1.73 $m^2$ or more and less than 45 ml/min/1.73 $m^2$.
(4) The drug according to any one of the above (1) to (3), wherein the primary glomerular disease or nephrosclerosis patient has a plasma concentration of 50 pg/ml or more on average 2 to 6 hours after administration of the sustained-release preparation comprising the compound represented by the general formula (I) as an active ingredient once after a meal at 120 μg as the compound represented by the general formula (I).
(5) The drug according to any one of the above (1) to (4), which is combined with an angiotensin-converting enzyme inhibitor as an active ingredient.
(6) The drug according to any one of the above (1) to (4), which is used to be administered simultaneously, separately, or sequentially with a different preparation comprising an angiotensin-converting enzyme inhibitor as an active ingredient.
(7) The drug according to any one of the above (1) to (4), which is combined preparations that are used to be administered simultaneously, separately, or sequentially in treatment or prophylaxis for preventing dialysis shift or renal death, the drug separately comprising the following two preparations (a) and (b):

(a) an oral sustained-release preparation comprising the compound represented by the general formula (I) as an active ingredient; and
(b) a preparation comprising an angiotensin-converting enzyme inhibitor as an active ingredient.

(8) The drug according to any one of the above (1) to (4), which is used in combination with an angiotensin-converting enzyme inhibitor.
(9) A drug for preventing dialysis shift or renal death, which is a sustained-release preparation comprising, as an active ingredient, a compound represented by the following general formula (I):

[Formula 2]

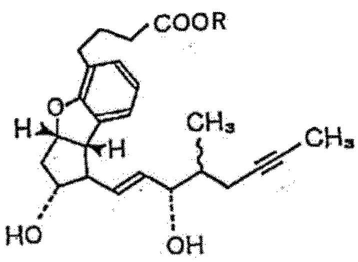

... (I)

wherein R represents hydrogen or a pharmacologically acceptable cation,
wherein the drug is used in such a manner that the compound represented by the general formula (I) is administered at 220 to 260 μg per day to a primary glomerular disease or nephrosclerosis patient with a nutritional disorder.

(10) The drug according to the above (9), wherein the compound represented by the general formula (I) is beraprost sodium.

(11) The drug according to the above (9) or (10), wherein the nutritional disorder is protein energy wasting (PEW) or a preliminary disease thereof.

(12) The drug according to any one of the above (9) to (11), wherein the nutritional disorder satisfies at least one of four constituent elements of PEW.

(13) The drug according to any one of the above (9) to (12), wherein the nutritional disorder satisfies a body mass index (BMI) of less than 23 or a serum albumin level of less than 3.8 g/dl.

(14) The drug according to any one of the above (9) to (13), wherein the nutritional disorder is cachexia, sarcopenia, or frailty.

(15) The drug according to any one of the above (9) to (14), wherein the target patient has a serum creatinine level of 2.0 mg/dl or more and less than 3.0 mg/dl.

(16) The drug according to any one of the above (9) to (14), wherein the target patient has an estimated glomerular filtration rate (eGFR), as calculated by the Chronic Kidney Disease Epidemiology Collaboration (CKD-EPI) equation, of 15 ml/min/1.73 $m^2$ or more and less than 45 ml/min/1.73 $m^2$.

(17) The drug according to any one of the above (9) to (16), wherein the primary glomerular disease or nephrosclerosis patient has a plasma concentration of 50 pg/ml or more on average 2 to 6 hours after administration of the sustained-release preparation comprising the compound represented by the general formula (I) as an active ingredient once after a meal at 120 μg as the compound represented by the general formula (I).

(18) The drug according to any one of the above (9) to (17), which is combined with an angiotensin-converting enzyme inhibitor as an active ingredient.

(19) The drug according to any one of the above (9) to (18), wherein the nutritional disorder is malnutrition.

(20) The drug according to any one of the above (9) to (19), wherein the nutritional disorder is represented by a total lymphocyte count of less than 1500/$mm^2$.

(21) A drug for preventing dialysis shift or renal death, which is a sustained-release preparation comprising a compound represented by the following general formula (I) as an active ingredient:

[Formula 3]

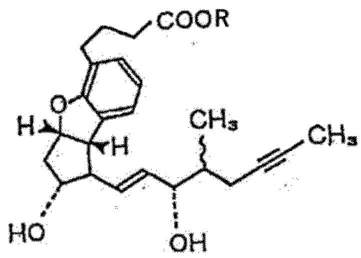

... (I)

wherein R represents hydrogen or a pharmacologically acceptable cation,

wherein the drug is used in such a manner that the compound represented by the general formula (I) is administered at 220 to 260 μg per day to a primary glomerular disease or nephrosclerosis patient with an estimated glomerular filtration rate (eGFR), as calculated by the Chronic Kidney Disease Epidemiology Collaboration (CKD-EPI) equation, of 15 ml/min/1.73 $m^2$ or more and less than 45 ml/min/1.73 $m^2$.

(22) The drug according to the above (21), wherein the compound represented by the general formula (I) is beraprost sodium.

(23) The drug according to the above (21) or (22), wherein the primary glomerular disease or nephrosclerosis patient has a plasma concentration of 50 pg/ml or more on average 2 to 6 hours after administration of the sustained-release preparation comprising the compound represented by the general formula (I) as an active ingredient once after a meal at 120 μg as the compound represented by the general formula (I).

(24) The drug according to any one of the above (21) to (23), which is combined with an angiotensin-converting enzyme inhibitor as an active ingredient.

(25) A drug for preventing dialysis shift or renal death, comprising a combination of a sustained-release preparation comprising a compound represented by the following general formula (I) as an active ingredient and an angiotensin-converting enzyme inhibitor as an active ingredient:

[Formula 4]

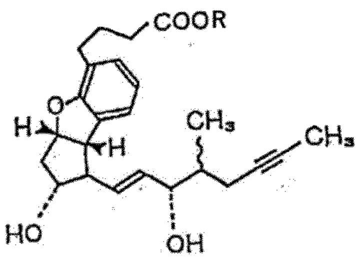

... (I)

wherein R represents hydrogen or a pharmacologically acceptable cation,
wherein the drug is used in such a manner that the compound represented by the general formula (I) is administered at 220 to 260 μg per day to a primary glomerular disease or nephrosclerosis patient.
(26) The drug according to the above (25), which is used to be administered simultaneously, separately, or sequentially with a different preparation comprising an angiotensin-converting enzyme inhibitor as an active ingredient.
(27) The drug according to the above (25) or (26), which is combined preparations to be used to be administered simultaneously, separately, or sequentially in treatment or prophylaxis for preventing dialysis shift or renal death, the drug separately comprising the following two preparations (a) and (b):

(a) an oral sustained-release preparation comprising the compound represented by the general formula (I) as an active ingredient; and
(b) a preparation comprising an angiotensin-converting enzyme inhibitor as an active ingredient.

(28) The drug according to any one of the above (25) to (27), which is used in combination with an angiotensin-converting enzyme inhibitor.

Advantageous Effects of Invention

The drug of the present invention can be administered to a primary glomerular disease or nephrosclerosis patient with a serum creatinine level of 2.0 mg/dl or more and less than 3.0 mg/dl before the start of treatment, thereby preventing dialysis shift or renal death.
Moreover, the combined preparation of the present invention can be administered to a chronic renal failure patient, thereby preventing dialysis shift or renal death.
Furthermore, the drug of the present invention can be administered to a primary glomerular disease or nephrosclerosis patient with an estimated glomerular filtration rate (eGFR), as calculated by the CKD-EPI equation, of 15 ml/min/1.73 $m^2$ or more and less than 45 ml/min/1.73 $m^2$, thereby preventing dialysis shift or renal death.
In addition, the drug of the present invention can be administered to a primary glomerular disease or nephrosclerosis patient with a complication of a nutritional disorder before the start of treatment, thereby preventing dialysis shift.

Brief Description of Drawings

[Figure 1] Figure 1 is a view showing the transition of event-free survival after administration of BPS or placebo to a Japanese patient group with a serum creatinine level of 2.0 mg/dl or more and less than 3.0 mg/dl before the start of administration. The event was dialysis shift.
[Figure 2] Figure 2 is a view showing the results of a group administered with BPS at 240 μg per day in a Japanese patient group with a plasma concentration of 50 pg/ml or more on average 2 to 6 hours after administration of BPS sustained-release tablets after a meal at 120 μg per dose when the event was dialysis shift.
[Figure 3] Figure 3 is a view showing the results of, among a 240-μg BPS-administered Japanese group and a 240-μg BPS-administered Asian group other than Japanese, a patient group combined with ACEI and a patient group not combined with ACEI when dialysis shift was set as an event.
[Figure 4] Figure 4 is a view showing the transition of event-free survival when 240 μg BPS or placebo was administered to Japanese chronic renal failure patients and Asian chronic renal failure patients other than Japanese with a body mass index (hereinafter, abbreviated as BMI) of less than 23 before the start of administration. The event was dialysis shift.

[Figure 5] Figure 5 is a view showing the transition of event-free survival when 240 μg BPS or placebo was administered to Japanese chronic renal failure patients with a BMI of less than 23 before the start of administration. The event was dialysis shift.
[Figure 6] Figure 6 is a view showing the transition of event-free survival after 240 μg BPS or placebo was administered to Japanese chronic renal failure patients with a serum albumin level of less than 3.8 g/dl before the start of administration. The event was dialysis shift.
[Figure 7] Figure 7 is a view showing the transition of event-free survival when 240 μg BPS or placebo was administered to Japanese chronic renal failure patients with a total lymphocyte count of less than 1500/$mm^3$ before the start of administration. The event was dialysis shift.

Description of Embodiments

The sustained-release preparation that can be used in the present invention comprises a compound represented by the following general formula (I):

[Formula 5]

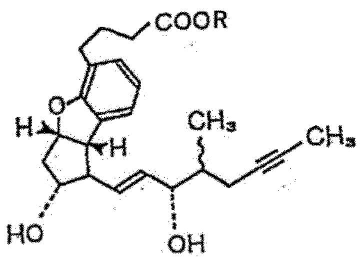

... (I)

wherein R represents hydrogen or a pharmacologically acceptable cation.
Examples of the pharmacologically acceptable cation include alkali metals and alkaline earth metals such as sodium, potassium, and calcium; amines typified by mono-, di-, or trimethylamine, methyl piperidine, mono-, di-, or triethanolamine, and lysine; basic amino acids; and the like. Of these, sodium and potassium are particularly preferably used.
Further, among the compounds represented by the formula (I), beraprost or pharmacologically acceptable salts thereof are preferably used. Of these, in addition to beraprost, BPS, which is a sodium salt of beraprost, or a potassium salt of beraprost is particularly preferably used.
BPS is composed of four stereoisomers, and its medicinal effect is mainly responsible for BPS-314d (sodium (+)-(1R,2R,3aS,8bS)-2,3,3a,8b-tetrahydro-2-hydroxy-1-[(E)-(3S,4S)-3-hydroxy-4-methyl-1-octen-6-ynyl]-1H-cyclopenta[b]benzofuran-5-butyrate) (Non-Patent Literature 12). Therefore, preparations containing only BPS-314d, which is an active ingredient of BPS, are also preferably used. Regarding the plasma concentration of BPS-314d when BPS is administered, both AUC (area under the blood concentration time curve; the area of the part surrounded by the curve (blood drug concentration-time curve) showing the time course of blood concentration and the horizontal axis (time axis)) and Cmax (maximum drug concentration) are known to be almost 1/4 (Non-Patent Literature 13). Therefore, when a preparation containing an active body of BPS (e.g., BPS-314d) alone is administered, the effective dose per day of BPS-314d is 55 to 65 μg, which is 1/4 the dose of BPS. Further, an active body of beraprost potassium (potassium (+)-(1R,2R,3aS,8bS)-2,3,3a,8b-tetrahydro-2-hydroxy-1-[(E)-(3S,4S)-3-hydroxy-4-methyl-1-octen-6-ynyl]-1H-cyclopenta[b]benzofuran-5-butyrate) is also particularly preferably used. The daily dose in this case is 57 to 67 μg.
As BPS preparations, immediate-release tablets are commercially available; however, the half-life in blood of BPS is as short as about 1 hour, and it is thus necessary to take them three or four times a day. Moreover, due to the rapid increase in plasma concentration, the frequency of side effects, such as flushing and headache, increases, so that the dose that can be administered has been limited. For this reason, as the drug of the present invention, beraprost or a pharmacologically acceptable salt thereof, preferably a BPS sustained-release preparation, particularly an oral sustained-release preparation, is used.
Sustained-release preparations are those that delay the release of active ingredients from preparations to reduce the number of doses, and keep the active ingredient concentration in the blood constant for a long period of time to avoid side effects, as described in the Pharmaceutical Glossary of the Pharmaceutical Society of Japan.
The sustained-release preparation of the present invention is defined as satisfying the dissolution behavior of the following dissolution test. That is, when a test is carried out at 100 rpm by the paddle method (however, using a

sinker) while taking one tablet of this preparation and using 50 ml of water containing 0.5 ml of Polysorbate 80 as a test liquid, the preparation has a 3-hour dissolution rate of 25 ± 10%, a 6-hour dissolution rate of 50 ± 10%, and a 10-hour dissolution rate of 70% or more; and the preparation preferably satisfies these dissolution rates within a pH range of 1.2 to 7.5.

By satisfying such conditions, preparations that show effectiveness by oral administration once or twice a day due to the sustainability etc. of the effective plasma concentration of BPS can be obtained.

The sustained-release preparation of the present invention is not particularly limited as long as it satisfies the above dissolution characteristics. For example, WO98/41210 and WO2004/103350 disclose BPS sustained-release preparations comprising a hydrogel base as a release control component of BPS. The BPS sustained-release preparations produced by these methods have already received manufacturing and marketing approval as therapeutic agents for pulmonary arterial hypertension, and have been widely clinically applied.

The BPS sustained-release preparation comprising a hydrogel base will be described in detail below. That is, the release control component refers to a substance that has the function of changing the release rate of BPS when being mixed in the preparation, and the mixing method etc. are not particularly limited. Examples of such release control components include so-called sustained-release bases that delay the release rate, buffers that suppress pH changes during release, such as pH changes in the gastrointestinal tract, to avoid the pH dependency of the release rate, solubilizing agents that improve the solubility of medicinal substances to stabilize the release rate with diffusion rate control, release promoters, and the like.

As the release control component, a hydrogel base is used, in terms of stably releasing a slight amount of BPS. When a hydrogel base is used as the release control component, even with a slight amount (about 0.1 to 10000 μg) of BPS, so-called zero-order release with very little variation over time in the release rate is possible.

As such hydrogel bases, known hydrogel bases can be used. The term "hydrogel" as used herein means a water-swellable polymer or a combination of two or more of such polymers. Such a hydrogel suitable for the object of the present invention is composed of a polymer substance that absorbs, when being brought into contact with water or another aqueous medium, water or the other medium and swells to some extent. Such absorption is reversible or irreversible, both of which are included in the scope of the present invention. As hydrogel bases, various polymer substances of natural and synthetic origin are known. In the present invention, desirable hydrogel bases are substantially linear polymers that are easy to control preparation production and the ability to control release by the molecular weight, that do not have a crosslinked structure as a covalent bond, and that do not have drug interaction and adsorption. Examples of such hydrogel bases include methylcellulose, hydroxypropylcellulose (hereinafter, abbreviated as HPC), hydroxypropylmethylcellulose (hereinafter, abbreviated as HPMC), polyethylene oxide (hereinafter, abbreviated as PEO), sodium carboxymethylcellulose, sodium alginate, sodium hyaluronate, and like water-soluble polymers, or mixtures of two or more of these.

As a preferred hydrogel base, HPC, HPMC, PEO, or a mixture of two or more of these is used. There are various types of hydrogel bases depending on the degree of viscosity, which are selected as appropriate depending on the intended purpose.

The content of the hydrogel base in the preparation is preferably 10 wt.% to the balance of BPS (when a buffer is contained, then the balance of BPS and the buffer), and more preferably 40 to 95 wt.%, based on the weight of the preparation.

In the present invention, buffers refer to substances that suppress pH changes during release, such as pH changes in the gastrointestinal tract, to avoid the pH dependency of the release rate, as described above. Examples thereof include those having a buffering action in the acidic region, those having a buffering action in the neutral region, and those having a buffering action in the basic region. It is preferable to suitably select one from these buffers depending on the physical properties of BPS. In the present invention, BPS has a weakly acidic carboxyl group; thus, it is desirable to control disassociation of the carboxyl group of BPS to keep solubility in the aqueous solvent constant in the hydrogel. Organic acids, amino acids, and inorganic salts are preferred. Examples of organic acids include citric acid, succinic acid, fumaric acid, tartaric acid, ascorbic acid, or salts thereof; examples of amino acids include glutamic acid, glutamine, glycine, aspartic acid, alanine, arginine, or salts thereof; and examples of inorganic salts include magnesium oxide, zinc oxide, magnesium hydroxide, phosphoric acid, boric acid, or salts thereof; and mixtures of one or two or more of these. In particular, in expectation of a long-term buffering effect, the buffer of the sustained-release preparation is preferably a poorly soluble buffer with a solubility in water of 15 wt.% or less. Examples thereof include succinic acid, fumaric acid, ascorbic acid, glutamine, glutamic acid, arginine, magnesium oxide, zinc oxide, magnesium hydroxide, boric acid, or salts thereof, and mixtures of two or more of these. Acidic buffers are more preferable because they reduce the dissolution rate and sustain the release by suppressing dissociation of the carboxyl group of BPS. Examples thereof include succinic acid, fumaric acid, ascorbic acid, glutamic acid, boric acid, or salts thereof, and mixtures of two or more of these. Such poorly soluble and acidic buffers are particularly preferable as the buffer of the sustained-release preparation because they suppress drug-releasing pH changes and also suppress changes in the release rate over time, so that a constant release rate can be maintained for a long period of time.

The buffer is used in an amount, for example, in the range of 0.1 to 30 wt.% of the weight per preparation. The preferred amount is 1 to 20 wt.%, and particularly preferably 1 to 10 wt.%.

The sustained-release preparation used in the present invention may contain, if necessary, available additives, such as excipients, lubricants, binders, stabilizers, and solubilizing agents. The additives are not particularly limited as long as they are pharmacologically acceptable. Examples of excipients include lactose, saccharose, sucrose, D-mannitol, sorbitol, xylitol, crystalline cellulose, corn starch, gelatin, polyvinyl pyrrolidone, dextran, polyethylene glycol (hereinafter, abbreviated as PEG) 1500, PEG 4000, PEG 6000, PEG 20000, polyoxyethylene polyoxypropylene glycol (PEP 101 (trademark) and Pluronic (trademark)), and the like. Further, examples of lubricants include magnesium stearate, calcium stearate, talc, and the like; examples of binders include hydroxypropylcellulose, hydroxypropylmethylcellulose, methylcellulose, stearic acid, propylene glycol, and the like; examples of stabilizers include butylhydroxytoluene, butylhydroxyanisole, ascorbic acid, propyl gallate, dibutylmethylphenol, sodium thiosulfate, and the like; and examples of solubilizing agents include cyclodextrin, polyethylene hydrogenated castor oil, polyethyleneglycol monostearate, and the like. The amounts of these additive mixed are selected as appropriate depending on the type and purpose thereof.

The additive content is not particularly limited, but is generally 0 wt.% to about the balance of BPS and the hydrogel base (when a buffer is contained, then the balance of BPS, the hydrogel base, and the buffer), and preferably 5 wt.% to about the balance of BPS and the hydrogel base (when a buffer is contained, then the balance of BPS, the hydrogel base, and the buffer), based on the weight per preparation.

The combination of BPS, the release control component, and the buffer in the sustained-release preparation used in the present invention is not particularly limited, and is, for example, a combination of BPS, polyethylene oxide, and a poorly soluble and acidic buffer such as fumaric acid or glutamic acid.

The form of the BPS sustained-release preparation containing the hydrogel base is not particularly limited; however, tablets are preferably used.

Moreover, the BPS sustained-release preparation that satisfies the dissolution characteristics described above can also be produced in the following manner. Specifically, WO2004/103350 discloses an oral sustained-release pharmaceutical composition comprising a plurality of granules having a particle size of 1000 μm or less, wherein the granules each comprise a BPS-containing nuclear granule and a coating agent, the coating agent is composed of at least two film layers including (1) a film layer containing a poorly water-soluble polymer substance and (2) a film layer containing a heat-meltable low-melting-point substance, and the nucleus granule is coated with the coating agent.

The amount of BPS mixed in the oral sustained-release pharmaceutical composition comprising the plurality of granules is not particularly limited as long as it is a therapeutically effective amount, and is, for example, in the range of 20 to 250 μg/preparation, and preferably in the range of 115 to 250 μg/preparation. The term "per preparation" as used herein means an amount of preparation orally administered at a time. The weight per preparation is not particularly limited, but is generally about 20 mg to 1000 mg.

The poorly water-soluble polymer substance that constitutes the film layer refers to a water-insoluble polymer substance that has film-forming ability and drug release control ability. The coating method or additives to be mixed are not particularly limited. Examples of such poorly water-soluble polymer substances include water-insoluble alkyl cellulose ether derivatives (e.g., ethyl cellulose and butyl cellulose), water-insoluble vinyl derivatives (e.g., polyvinyl acetate and polyvinyl butyrate), and water-insoluble acrylic polymer derivatives (e.g., acrylic acid-methacrylic acid copolymers), and mixtures of two or more of these. Examples of preferred poorly water-soluble polymer substances include acrylic acid-methacrylic acid copolymers.

The heat-meltable low-melting-point substance that constitutes the film layer refers to a heat-meltable substance that has a relatively low melting point, preferably 70°C or lower, and more preferably from room temperature to 70°C, and that has release control ability. The coating method and the additives to be mixed are not particularly limited. Examples of such heat-meltable low-melting-point substances include higher fatty acids (e.g., stearic acid, capric acid, lauric acid, myristic acid, and palmitic acid), higher alcohols (e.g., stearyl alcohol, myristyl alcohol, lauryl alcohol, and cetyl alcohol), higher fatty acid glycerol esters (e.g., glycerol palmitate oleate, glycerol monooleate, glycerol monostearate, glycerol monomyristate, glycerol monobehenate, glycerol trimyristate, and glycerol tribehenate), waxes (e.g., carnauba wax), saturated hydrocarbons (e.g., paraffin), and mixtures of two or more of these. Examples of preferred heat-meltable low-melting-point substances include cetyl alcohol, stearic acid, glycerol palmitate oleate, glycerol monooleate, glycerol monostearate, glycerol monomyristate, glycerol monobehenate, glycerol tristearate, glycerol trimyristate, and glycerol tribehenate.

The weight ratio of (1) the film layer containing a poorly water-soluble polymer substance and (2) the film layer containing a heat-meltable low-melting-point substance in the film layer, and the coverage of the film layer in the granule are not particularly limited, and are determined as appropriate depending on the drug used, the effective dosage, and the like. In general, the ratio thereof may be within the range of 1:9 to 9:1, and preferably within the range of 3:7 to 7:3.

The film layer may contain pharmacologically acceptable additives. Examples of additives include propylene glycol, polyethylene glycol (hereinafter, abbreviated as PEG) 1500, PEG 4000, PEG 6000, PEG 20000, polyoxyethylene polyoxypropylene glycol (PEP 101 (trademark) and Pluronic (trademark)), glycerol, triethyl citrate, tributyl citrate, triacetin,

sodium lauryl sulfate, sorbitol, polyvinylpyrrolidone, Polysorbate 80, and like plasticizers. The effective amount of pharmaceutical plasticizer currently available on the market varies between 1 to 30% of the total dry weight of the coating material.

Examples of brittleness inducers, which are additives that reduce the elasticity of films forming the coatings, include talc, magnesium stearate, calcium stearate, Aerosil, and titanium oxide. The effective amount of brittleness inducer varies depending on the type of brittleness inducer used. For example, when talc is used, the effective amount is 10 to 70%, when Aerosil, 1 to 40%, and when magnesium stearate, 1 to 70%. Here, all percentages are based on the total dry weight of the coating material.

Additives that can be mixed into the BPS-containing nuclear granules are not particularly limited as long as they are pharmacologically acceptable.

Examples of preferred additives include binders, excipients, stabilizers, solubilizing agents, and buffers.

Examples of binders include hydroxypropylcellulose, hydroxypropylmethylcellulose, methylcellulose, stearic acid, and propylene glycol. Examples of excipients include lactose, saccharose, sucrose, D-mannitol, sorbitol, xylitol, crystalline cellulose, corn starch, gelatin, polyvinylpyrrolidone, dextran, PEG 1500, PEG 4000, PEG 6000, PEG 20000, and polyoxyethylene polyoxypropylene glycol (PEP 101 (trademark) and Pluronic (trademark)). BPS-containing nuclear granules can be prepared by coating existing spherical granules, such as Nonpareil (trademark) (saccharose), Suglets (trademark) (saccharose), or Ethispheres (trademark) (crystalline cellulose), with a pharmaceutically active substance together with a binder. Alternatively, BPS-containing nuclear granules can be produced by mixing BPS with an excipient, and granulating the mixture into spherical granules. Examples of stabilizers include butylhydroxytoluene, butylhydroxyanisole, ascorbic acid, propyl gallate, dibutylmethylphenol, sodium thiosulfate, and titanium oxide. The effective compounding amount varies depending on the pharmaceutically active substance. Examples of solubilizing agents include cyclodextrin, polyethylene hydrogenated castor oil, polyethylene glycol monostearate, poloxamer, and Polysorbate 80. Examples of buffers include alkaline reactants such as MgO, and acidic reactants such as organic acids (e.g., citric acid and tartaric acid).

The particle size of the granules is 1000 μm or less, preferably 100 to 850 μm, and more preferably 300 to 750 μm.

The oral sustained-release pharmaceutical composition comprising the plurality of granules is composed of a plurality of granular particles having a particle size of 1000 μm or less, each of which has a sustained-release function. By controlling the particle size within the above-mentioned range, it is possible to maintain stable release in the lower part of the gastrointestinal tract. The final form thereof is not particularly limited, but may be a form that can be orally administrated. Examples thereof include tablets, granules, fine granules, capsules, suspensions, and the like.

Since the drug of the present invention is a pharmaceutically stable preparation with excellent sustainability, oral administration once or twice a day results in stable medicinal properties for a long period of time and excellent bioavailability, and it is easy to take.

Examples of sustained-release preparations for oral administration include single-unit and multiple-unit sustained-release preparations. Many of single-unit preparations gradually release drugs while the dosage form is maintained in the gastrointestinal tract. Examples of single-unit preparations include wax matrix, gradumet, repetab, lontab, spantab, and the like. As for multiple-unit preparations, administered tablets or capsules are rapidly distinguished to release granules, and the released granules show sustained-release properties. Examples of multiple-unit preparations include spacetab, spansule, granule, and the like. Further, in terms of release control mechanism, they are divided into reservoir preparations and matrix preparations. Reservoir preparations are obtained by coating drug-containing tablets or granules with polymer coatings, and the drug release rate is determined by the properties and thickness of the coating. Repetab, spacetab, spansule, and granule belong to reservoir preparations. Matrix preparations are obtained by dispersing drugs in bases such as polymers or waxes, and the release rate is determined by the diffusion rate of drug molecules in the matrix. Wax matrix, gradumet, lontab, spantab, etc., belong to matrix preparations. Various sustained-release preparations can be used, regardless of the method of sustained-release, as long as they have the release characteristics described above.

For the drug of the present invention, the sustained-release preparation comprising a compound represented by the formula (I) as an active ingredient is administered once or twice so that the dose of the compound represented by the formula (I) is 220 to 260 μg per day.

The sustained-release preparations comprising the compound represented by the formula (I) as an active ingredient are commercially available as Careload (trademark) LA Tablets 60 μg (Toray Industries, Inc.) and Berasus (trademark) LA Tablets 60 μg (Kaken Pharmaceutical Co., Ltd.) as BPS-containing sustained-release preparations. Therefore, when 2 tablets of Careload (trademark) LA Tablets 60 μg (Toray Industries, Inc.) or Berasus (trademark) LA Tablets 60 μg (Kaken Pharmaceutical Co., Ltd.) are administered per dose twice a day (4 tablets in total), 240 μg BPS per day is supposed to be administered.

Further, as sustained-release preparations that can be used in the present invention, compared to sustained-release preparations that have been approved for manufacturing and marketing in Japan as Careload (trademark) LA Tablets 60 μg (Toray Industries, Inc.) or Berasus (trademark) LA Tablets 60 μg (Kaken Pharmaceutical Co., Ltd.),

preparations for which bioequivalence is shown by dissolution behavior, clinical pharmacokinetic studies, etc., according to the "Guideline for Bioequivalence Studies of Generic Products," SUPAC-MR (Modified Release Solid Oral Dosage Forms), or the like are particularly preferably used.

The drug of the present invention can be administered to a primary glomerular disease or nephrosclerosis patient with a serum creatinine level of 2.0 mg/dl or more and less than 3.0 mg/dl before the start of treatment and/or an estimated glomerular filtration rate (eGFR), as calculated by the CKD-EPI (Chronic Kidney Disease Epidemiology Collaboration) equation, of 15 ml/min/1.73 $m^2$ or more and less than 45 ml/min/1.73 $m^2$ before the start of treatment, thereby preventing dialysis shift or renal death.

Non-Patent Literature 3 has reported that as a result of long-term follow-up when 20 μg BPS immediate-release tablets were administered three times a day to chronic renal failure patients, a patient group with a serum creatinine level of 2.8 mg/dl or more before the start of BPS administration shifted to dialysis within 24 months, and that the effect of BPS was not observed when the serum creatinine level was 2.2 mg/dl or more. However, in the present invention, the application target can be limited to primary glomerular disease or nephrosclerosis patients, thereby preventing dialysis shift or renal death. Further, dialysis shift or renal death can also be prevented in patients with a serum creatinine level of 2.2 mg/dl or more, for which no effect has been confirmed in Non-Patent Literature 3.

The dialysis in the present specification includes peritoneal dialysis, in addition to blood dialysis.

The drug of the present invention shows practically excellent effects for, among the primary glomerular disease or nephrosclerosis patients mentioned above, patients with a plasma concentration of 50 pg/ml or more on average 2 to 6 hours after administration of a BPS sustained-release preparation at 120 μg as BPS once after a meal.

Since the pharmacological action of BPS is observed depending on the concentration of BPS, it is estimated that a higher effect will be obtained as the plasma concentration at the time of administration of BPS is higher. From the relationship between Cmax and AUC (when Cmax is 214.7 ± 89.1 pg/ml, AUC is 1225 ± 343 pg·hr/ml, which is about 5.7 times) at the time of administration of 240 μg BPS sustained-release tablets, AUC estimated when Cmax of the sustained-release tablets is 50 pg/ml is 286 pg·hr/ml, and due to administration twice a day, AUC per day is 572 pg·hr/ml.

On the other hand, regarding Cmax and AUC when 20 μg immediate-release tablets, for which no sufficient dialysis delay effect has been confirmed, are administered to a healthy subject, 1) when 40 μg BPS immediate-release tablets are orally administered daily to a human twice a day after a meal, Cmax on day 7 at the time of final administration is 242.2 ± 81.4 pg/ml, and AUC is 550 ± 148 pg·hr/ml (interview form of Careload Tablets), and 2) linearity is observed between the dosage of BPS immediate-release tablets, Cmax, and AUC (Non-Patent Literature 12). Thus, Cmax is 121 pg/ml and AUC is about 275 pg·hr/ml. Since immediate-release tablets are generally administered three times a day, AUC per day is assumed to be about 825 pg·hr/ml. Further, it has been reported that both Cmax and AUC are elevated in renal damage patients compared to healthy subjects (Non-Patent Literature 13). Therefore, Cmax and AUC were considered to be even higher when 20 μg immediate-release tablets were administered to a chronic renal damage patient.

In light of the above, it could not be predicted from the conventional finding that the significant effect of beraprost sustained-release tablets on prevention of dialysis shift or renal death in patients with a plasma concentration of 50 pg/ml or more on average 2 to 6 hours after administration (AUC per day is 570 pg·hr/ml or more), which was around Cmax at the time of administration of BPS sustained-release tablets, was observed in Cmax and AUC lower than those in the case of administration of 20 μg immediate-release tablets three times a day.

In the present specification, regarding the phrase "a plasma concentration of 50 pg/ml or more on average 2 to 6 hours after administration of a BPS sustained-release preparation at a dose of 120 μg as BPS once after a meal," the plasma concentration may be measured once or more times 2 to 6 hours after administration, and the average of the measured values may be 50 pg/ml or more; or "administration at 120 μg once after a meal" may be performed once or more times, and the average of the measured values may be 50 pg/ml or more. These may also be combined.

Moreover, it is known that when BPS is administered to a human, the Cmax of BPS-314d, which is the essence of the activity of BPS, is 1/4 that of BPS (Non-Patent Literature 13). Therefore, it is possible to replace the particularly effective plasma concentration of BPS in the present invention, i.e., 50 pg/ml or more, by the plasma concentration of BPS-314d, i.e., 12.5 pg/ml or more. In particular, when a preparation containing BPS-314d alone is administered, it is necessary to define it by the plasma concentration of BPS-314d.

The drug of the present invention exhibits particularly excellent effects for, among primary glomerular disease or nephrosclerosis patients, patients who take ACEI. Further, the drug of the present invention is particularly effectively used for patients with a serum creatinine level of 2.0 mg/dl or more and less than 3.0 mg/dl before administration, or patients with an eGFR, as calculated by the CKD-EPI equation, of 15 ml/min/1.73 $m^2$ or more and less than 45 ml/min/1.73 $m^2$.

Examples of the ACEI include captopril, enalapril, benazepril, imidapril, lisinopril, perindopril, ramipril, moexipril, fosinopril, and quinapril. The usage and dosage of these ACEIs may follow the usage and dosage of each ACEI approved as an antihypertensive agent. Moreover, when ACEI is contained as an active ingredient, compound drugs of ARB, a calcium antagonist, a beta blocker, and various diuretics may be used.

The present invention provides a drug that is combined preparations used to be administered simultaneously,

separately, or sequentially in treatment or prevention for preventing dialysis shift or renal death, the drug separately comprising the following two preparations (a) and (b):

(a) an oral sustained-release preparation comprising a compound represented by the general formula (I) as an active ingredient, for administering the compound represented by the general formula (I) at 220 to 260 μg per day; and
(b) a preparation comprising ACEI as an active ingredient.

The drug of the present invention, which separately comprises two preparations, can be administered to chronic renal failure patients, thereby preventing dialysis shift or renal death.

The drug of the present invention is particularly effectively used for primary glomerular disease or nephrosclerosis patients with nutritional disorders.

The drug of the present invention is particularly effective for malnutrition among nutritional disorders. Malnutrition is generally defined by the serum protein mass, body size, muscle mass, nutritional intake, and the like; however, various standards have been proposed, and it is thus not always limited to one standard.

The present invention is particularly effectively used for cases that satisfy the criteria of PEW as a nutritional disorder, which are characteristic indicators of renal damage patients, and for cases that satisfy the elements constituting PEW. Specifically, any one of the four elements constituting PEW, i.e., "serum chemistry," "body mass," "muscle mass," and "dietary intake," may be satisfied, and further one parameter in each element may be satisfied. Needless to say, some of the elements may be satisfied; rather, the effect of BPS is more clearly observed in typical cases having some of the elements.

Further, the drug of the present invention is particularly effective for patients with nutritional disorders who have a serum creatinine level of 2.0 mg/dl or more and less than 3.0 mg/dl, and patients with nutritional disorders who have an estimated glomerular filtration rate, as calculated by the CKD-EPI equation, of 15 ml/min/1.73 $m^2$ or more and less than 45 ml/min/1.73 $m^2$.

In addition, the drug of the present invention is also effectively used for chronic renal failure patients with a complication of pre-cachexia or cachexia, both of which are more serious PEW. Further, the drug of the present invention is extremely useful for chronic renal failure patients with a complication of sarcopenia or frailty, both of which occur as a result of the progress of nutritional disorder.

The treatment using the drug of the present invention does not result in a decrease in the body weight or muscle mass during treatment with SGLT2 inhibitors, and can therefore be effectively used for chronic renal failure patients with PEW or cachexia, further with a complication of sarcopenia or frailty.

In the primary glomerular disease or nephrosclerosis of the present invention, there is no particular problem even if there is a complication of diabetes.

(Method for calculating eGFR)

The eGFR values in the present invention are calculated by the CKD-EPI equation and described in Non-Patent Literature 14. Specifically, the eGFR values are expressed as follows. That is, when [Cr] ml/dl represents serum creatinine, and [Age] represents age, eGFR is calculated by the following Equation 1 for males, and the following Equation 2 for females:

$$\text{eGFR (ml/min/1.73 m}^2) = 141 \times ([\text{Cr}]/0.9)^{-1.209} \times 0.993^{[\text{Age}]} \ldots \text{Equation 1}$$

$$\text{eGFR (ml/min/1.73 m}^2) = 144 \times ([\text{Cr}]/0.9)^{-1.209} \times 0.993^{[\text{Age}]} \ldots \text{Equation 2}$$

In the CKD-EPI equation, separately, correction coefficients are applied so as to match the measured values using iothalamate and inulin in various countries including Japan. It is expected that it will be proposed in the future. However, in the present invention, it is preferable to use the above estimate equations based on the original document. For serum creatine used in the calculation of the CKD-EPI equation, values measured by the enzymatic method are used in principle. Values measured by the Jaffe method need to be corrected.

(Method for measuring serum creatinine levels)

Serum creatinine levels are measured by the enzymatic method. Specifically, in addition to Cygnus Auto CRE (Shino-Test Corporation), L-type Wako CRE·M (FUJIFILM Wako Pure Chemical Corporation), Pureauto S CRE-N (Sekisui Medical Co., Ltd.), Serotec CRE-N (Serotec Co., Ltd.), Aqua-auto Kainos CRE-III plus (Kainos Laboratories, Inc.),

and Shikarikid-N CRE (Kanto Chemical Co., Inc.), all of which are sold as clinical test drugs, are used; however, any clinical test drugs that use the enzymatic method can be used without any particular limitation.

When the enzymatic method is compared to the conventionally used Jaffe method, it is cautioned that the Jaffe method, which has lower specificity, results in 0.2 mg/ml higher values (Non-Patent Literature 15). Therefore, when creatinine levels measured by the Jaffe method are used, 0.2 mg/ml is subtracted to convert them to values measured by the enzymatic method.

(Method for measuring plasma BPS concentration)

The plasma BPS concentration is quantified by the GC-MS method, LC-MS method, LC-MS-MS method, or the like; however, any validated method can be used.

For the analysis, the full analysis set and Intention-To-Treat (hereinafter, abbreviated as ITT) defined in Non-Patent Literature 5 were used, and the hazard ratio (hereinafter, abbreviated as HR) of the drug of the present invention and placebo was calculated by the Cox proportional hazard model. A lower HR value indicates a higher effectiveness of the drug of the present invention.

Examples

Next, the present invention will be described in more detail while showing Examples and Comparative Examples; however, the present invention is not limited by these examples. The measurement methods performed in the following Examples and Comparative Examples are shown below.

(Method for calculating eGFR)

eGFR in the present invention was calculated using the Equations 1 and 2 described above.

(Method for measuring serum creatinine levels)

Serum creatinine levels were measured by the enzymatic method in SRL, Inc.

(Method for measuring plasma BPS concentration)

The plasma BPS concentration was quantified by the LC-MS/MS method in Toray Research Center, Inc.

(Example 1)

A BPS sustained-release tablet, TRK-100STP (the same preparation as Careload (trademark) LA Tablets 60 $\mu$g (Toray Industries, Inc.)), which is a gel-matrix sustained-release preparation containing 60 $\mu$g BPS and, as additives, polyethylene oxide 5000K, Macrogol 6000, L-glutamic acid, and magnesium stearate, was produced together with placebo tablets at Mishima Plant of Toray Industries, Inc. The *in vitro* dissolution behavior from TRK-100STP was measured in the following manner. Specifically, a test was carried out at 100 rpm by the paddle method (however, using a sinker) while taking one tablet of this preparation and using 50 ml of distilled water containing 0.5 ml of Polysorbate 80 as a test liquid. At this time, the 3-hour dissolution rate of BPS was 25%, the 6-hour dissolution rate was 50%, and the 10-hour dissolution rate was 83%. Further, when a dissolution test was carried out by the same protocol using Japanese Pharmacopoeia 1st liquid (pH: 1.2) and Japanese Pharmacopoeia 2nd liquid (pH: 6.8) in place of water, equivalent dissolution rates were obtained.

A data set obtained in the CASSIOPEIR trial according to the protocol described in Non-Patent Literature 5 was used. Summarizing the protocol of the CASSIOPEIR trial, the targets are chronic renal failure patients with primary glomerular disease and nephrosclerosis as primary diseases, and a BPS sustained-release tablet, TRK-100STP (the same preparation as Careload (trademark) LA Tablets 60 $\mu$g (Toray Industries, Inc.)), is administered at 120 $\mu$g or 240 $\mu$g as BPS per day in two divided doses, morning and evening. Further, this trial was a multicenter, randomized, placebo-controlled, double-blind comparative trial, and carried out in Japan, China, South Korea, Taiwan, Hong Kong, Malaysia, and Thailand.

The duration of drug administration was 2 to 4 years, and the number of patients underwent randomization was 892, and the trial was conducted in 160 sites. Further, the ITT population was the primary analysis target population. The primary endpoint was the time to occurrence of the renal composite endpoint defined by doubling of serum creatinine or end-stage renal disease. The guidelines (2013) of the Japanese Society for Dialysis Therapy were used as a reference for determining the time to shift to dialysis. Specifically, according to the policy that "even with adequate conservative

treatment, progressive deterioration of renal function is observed, and the need arises when GFR < 15 ml/min/1.73 $m^2$. However, the introduction of actual blood dialysis is determined by comprehensively judging the symptoms of renal failure, activity in daily life, and nutritional status, and a decision is made when these cannot be avoided except by dialysis therapy," and finally based on the judgment of the investigator, the time to shift to dialysis and the time to reach renal death were determined. The term "dialysis" as used herein includes peritoneal dialysis, in addition to blood dialysis.

Furthermore, in the CASSIOPEIR trial, in the Japanese population who had been confirmed to have a high drug compliance rate from the description of the case report form and the measurement results of plasma concentrations, a patient group with a serum creatinine level of 2.0 mg/dl or more and less than 3.0 mg/dl before the start of administration showed an HR of 0.51 and a P value of 0.0429, thus confirming the effect of preventing dialysis shift (Figure 1). Moreover, when the event was the time to renal death defined by dialysis shift or transplantation, the HR was 0.54 and the P value was 0.0624, which was below 0.1. A strong tendency similar to the case of dialysis shift was observed. Further, patients with a serum creatinine level of more than 2.2 mg/dl and less than 3.0 mg/dl before the start of administration showed an HR of 0.51 and a P value of 0.0495 when dialysis shift was set as an event. A significant effect of prolonging the time to dialysis shift was observed. In addition, in the Japanese population in the CASSIOPEIR trial, a patient group with an eGFR, as calculated by the CKD-EPI equation, of 15 ml/min/1.73 $m^2$ or more and less than 45 ml/min/1.73 $m^2$ before the start of administration showed an HR of 0.64 and a P value of 0.0691 when dialysis shift was set as an event. A tendency of prolonging the time to dialysis shift was observed.

(Comparative Example 1)

In the CASSIOPEIR trial, Japanese patients with a serum creatinine level of 3.0 mg/dl or more before the start of treatment showed an HR of 0.91 and a P value of 0.7219 when dialysis shift was set as an event. No effect of TRK-100STP administration was observed. Similarly, Japanese patients with an eGFR, as calculated by the CKD-EPI equation, of less than 15 ml/min/1.73 $m^2$ before the start of treatment showed an HR of 1.05 when dialysis shift was set as an event. No effect of TRK-100STP administration was observed at all.

(Example 2)

Of all the Japanese patients participating the CASSIOPEIR trial, in patients who was confirmed to have a plasma concentration of 50 pg/ml or more on average 2 to 6 hours after administration of BPS sustained-release tablets at 120 μg as BPS per dose after a meal, the effect of administration at 240 μg per day was examined while setting the time to dialysis shift as an event. As a result, HR = 0.63 and P value = 0.0315, indicating that dialysis shift was delayed by administration of 240 μg BPS (Figure 2). A similar tendency was also observed when the event was renal death. In Example 1 and this Example of the present specification, the data of the Japanese population with a high drug compliance rate were used for analysis; however, the application target is not limited to Japanese patients as long as drug compliance is maintained. Similar effects will be obtained from patients of China, South Korea, Taiwan, Hong Kong, Malaysia, Thailand, and other Asian countries, as well as other countries. For example, for Chinese and Thailand cases in the CASSIOPEIR trial, in patients with a plasma concentration of 50 pg/ml or more on average 2 to 6 hours after administration, who were assumed to take medication more reliably, the HR was 0.74 in both cases when dialysis shift was set as an event, thus indicating effectiveness almost equal to that of the Japanese partial population.

Further, in patient groups for which the above BPS concentration was confirmed, a patient group with an eGFR, as calculated by the CKD-EPI equation, of 15 ml/min/1.73 $m^2$ or more and less than 45 ml/min/1.73 $m^2$, who corresponded to CKD stages 3b and 4 as CKD stages before the start of treatment, showed HR = 0.59 and P value = 0.0368. The effect of minimizing the time to dialysis shift was more significant. Moreover, a patient group with a serum creatinine level of less than 3 mg/dl showed HR = 0.46 and P value = 0.0251. The effect of minimizing the time to dialysis shift was further significant. A similar tendency was also observed when renal death was set as an event.

(Comparative Example 2)

In Example 2, in patients who did not show a plasma concentration of 50 pg/ml or more after administration of BPS, the time to dialysis shift was not prolonged by the administration of BPS sustained-release tablets at 240 μg per day in the Chinese case and further in the Thailand case, in addition to the Japanese case. The prolongation of the time to renal death was also the same.

(Example 3)

In the CASSIOPEIR trial, when the time to dialysis shift or renal death was set as an event in all of the subjects who used BPS sustained-release tablets in combination with ACEI (lisinopril, temocapril, delapril, perindopril, benazepril,

enalapril, captopril, quinapril, fosinopril, imidapril, or a hydrochloride thereof, cilazapril, trandolapril, quinapril, ramipril, temocapril, or a hydrochloride thereof, or trandolapril), the HR and P value of groups administered with BPS at 240 μg per day were analyzed by the Cox Hazard model.

Among the groups administered with BPS at 240 μg per day, a patient group who used ACEI in combination (except for patients who further used ARB in combination) showed HR = 0.60 and P value = 0.0994 (Figure 3). The P value was below 0.1, strongly suggesting that the combined use of 240 μg BPS and ACEI prolonged the time to dialysis shift.

Further, when the patient group was limited to Japanese patients, HR = 0.49 and P value = 0.0882, suggesting a further significant effect of prolonging the time to dialysis shift by the combined use.

(Comparative Example 3)

In the CASSIOPEIR trial, in the case of patient groups who used ARB (telmisartan, valsartan, losartan, irbesartan, candesartan, olmesartan, or eprosartan) in combination, but did not use ACEI in combination, the hazard ratio and P value of the 240 μg BPS administration group when the time to dialysis shift was set as an event were HR = 0.88 and P value = 0.5401. The Japanese partial population showed HR = 0.79 and P value = 0.3597. In the ARB combined use group, no effect of prolonging the time to dialysis shift by the administration of BPS at 240 μg per day was observed.

(Comparative Example 4)

Further, in the case of using, in combination, a calcium antagonist (amlodipine, aranidipine, azelnidipine, barnidipine, benidipine, cilnidipine, clevidipine, efonidipine, felodipine, isradipine, lacidipine, lercanidipine, manidipine, nicardipine, nifedipine, nilvadipine, nimodipine, nisoldipine, nitrendipine, nitrepin, pranidipine, fendiline, gallopamil, verapamil, diltiazem, Micamlo Combination, Sevikar, ecroforge, or amlodipine) widely used as an antihypertensive agent, when BPS sustained-release tablets were administered at 240 μg per day, HR = 0.85 and P value = 0.3718 when the time to dialysis shift was set as an event. No effect of prolonging the time to dialysis shift was observed.

(Example 4)

As an indicator of patients with malnutrition, a BMI of less than 23 (see Table 2 below), which is one of the diagnostic criteria for PEW, was used. In the CASSIOPEIR trial, a patient group with a BMI of less than 23 showed HR = 0.66 and P value = 0.0411 when dialysis shift was set as an event. A significant effect of preventing dialysis shift by 240-μg administration was confirmed (Figure 4).

[Table 2]

| Diagnostic criteria | |
|---|---|
| Serum chemistry | Serum albumin<3.8g/dl |
| | Serum prealbumin (transthyretin)<30mg/dl |
| | Serum cholesterol < 100mg/dl |
| Body mass | BMI<23 |
| | Unintended loss of body weight: 5%/3 months or 10%/6 months |
| | Body-fat percentage< 10% |
| Muscle mass | Muscle wasting: loss of muscle mass: 5%/3 months or 10%/6 months 10% or more decrease in arm muscle circumference |
| Dietary intake | Dietary protein intake < 0.6 g/kg/day continues for at least 2 months |
| | Dietary energy intake < 25 kcal/kg/day continues for at least 2 months |

Further, Japanese patients with a BMI of less than 23 showed HR = 0.38 and P value = 0.0048, thus confirming a further significant effect of preventing dialysis shift (Figure 5). Moreover, in the BPS sustained-release tablet administration group and the placebo group, no significant change in BMI was observed before and after administration. It was confirmed that the above dialysis shift prevention effect was not caused by the change in BMI during the trial period. This finding was also observed when the event was renal death defined by dialysis shift or transplantation.

(Comparative Example 5)

Of all of the patients participating the CASSIOPEIR trial, patients with a BMI of 23 or more showed HR = 1.38 and P value = 0.0754 when dialysis shift was set as an event. The time to dialysis shift was not prolonged by the BPS sustained-release tablets. Even in the case of the Japanese population, HR = 1.06 and P value = 0.8266; no effect of the BPS sustained-release tablets was observed.

(Example 5)

As an indicator of patients with malnutrition, a serum albumin level of less than 3.8 g/dl (Table 2), which is one of the diagnostic criteria for PEW, was used. Among the Japanese patients participating the CASSIOPEIR trial, patients with a serum albumin level of less than 3.8 g/dl showed HR = 0.50 and P value = 0.1567 when dialysis shift was set as an event. There was a tendency of preventing dialysis shift by 240-$\mu$g administration (Figure 6). Further, in the BPS sustained-release tablet administration group and the placebo group, no change in serum albumin levels was observed before and after administration. It was confirmed that the above dialysis shift prevention effect was not caused by the change in serum albumin levels.

Among the above patients, a patient group with an eGFR, as calculated by the CKD-EPI equation, of 15 ml/min/1.73 $m^2$ or more and less than 45 ml/min/1.73 $m^2$, who corresponded to CKD stages 3b and 4 as CKD stages before the start of treatment, showed HR = 0.43 and P = 0.0273 when dialysis shift was set as an event, indicating further significant effectiveness of this drug. This finding was also observed when the event was renal death defined by dialysis shift or transplantation.

(Comparative Example 6)

Among the Japanese patients participating the CASSIOPEIR trial, patients with a serum albumin level of 3.8 g/dl or more showed HR = 0.78 and P value = 0.2656 when dialysis shift was set as an event. The HR was close to 1, and the time to dialysis shift was not prolonged at all by the BPS sustained-release tablets.

(Example 6)

Among the Japanese patients participating the CASSIOPEIR trial, patients with a total lymphocyte count of less than 1500/$mm^3$ showed HR = 0.62 and P value = 0.0689 when dialysis shift was set as an event. There was a tendency of preventing dialysis shift by 240-$\mu$g administration (Figure 7). Further, in the BPS sustained-release tablet administration group and the placebo group, no change in the lymphocyte count was observed before and after administration. It was confirmed that the above dialysis shift prevention effect was not caused by the change in the lymphocyte count itself.

Among the above patients, a patient group with an eGFR, as calculated by the CKD-EPI equation, of 15 ml/min/1.73 $m^2$ or more and less than 45 ml/min/1.73 $m^2$, who corresponded to CKD stages 3b and 4 as CKD stages before the start of treatment, showed HR = 0.52 and P value = 0.0383. The effect of prolonging the time to dialysis shift was further significant. This finding was also observed when the event was renal death defined by dialysis shift or transplantation.

(Comparative Example 7)

Among the Japanese patients participating the CASSIOPEIR trial, patients with a total lymphocyte count of 1500/$mm^3$ or more showed HR = 0.83 and P value = 0.5822 when dialysis shift was set as an event. The HR was close to 1, and the time to dialysis shift was not prolonged at all by the BPS sustained-release tablets.

(Example 7)

Among the Japanese patients participating the CASSIOPEIR trial, patients with a BMI of less than 23 and a serum albumin level of less than 3.8 g/dl showed HR = 0.29 when dialysis shift was set as an event. As described in Examples 4 and 5, the Japanese patient group with a BMI of less than 23 showed HR = 0.38, and the Japanese patient group with a serum albumin level of less than 3.8 g/dl showed HR = 0.50; thus, the effect of BPS was observed more significantly in cases with some of the constituent elements of PEW, rather than cases with one of the constituent elements of PEW. This finding was also observed when the event was renal death defined by dialysis shift or transplantation.

(Example 8)

Among the Japanese patients participating the CASSIOPEIR trial, a patient group with a total lymphocyte count of less than 1500/mm$^3$ and a serum albumin level of less than 3.8 g/dl showed HR = 0.23 and P value = 0.0404 when dialysis shift was set as an event. Compared to patients who satisfied only one criterion, i.e., a BMI of less than 23, a serum albumin level of less than 3.8 g/dl, or a total lymphocyte count of less than 1500/mm$^3$, as the elements constituting nutritional disorders described in Examples 4, 5, and 6, in patients with more significant nutritional disorders having some of these constituent elements, a significant effect was observed in the group administered with BPS sustained-release tablets at 240 μg per day.

## Claims

1. A drug for preventing dialysis shift or renal death, which is a sustained-release preparation comprising, as an active ingredient, a compound represented by the following general formula (I):

[Formula 1]

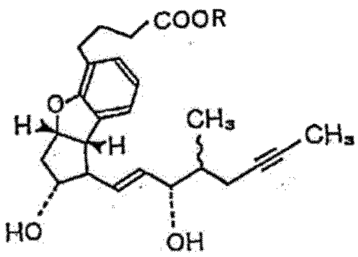

... (I)

wherein R represents hydrogen or a pharmacologically acceptable cation,
wherein the drug is used in such a manner that the compound represented by the general formula (I) is administered at 220 to 260 μg per day to a primary glomerular disease or nephrosclerosis patient with a serum creatinine level of 2.0 mg/dl or more and less than 3.0 mg/dl.

2. The drug according to claim 1, wherein the compound represented by the general formula (I) is beraprost sodium.

3. The drug according to claim 1 or 2, wherein the primary glomerular disease or nephrosclerosis patient has an estimated glomerular filtration rate (eGFR), as calculated by the Chronic Kidney Disease Epidemiology Collaboration (CKD-EPI) equation, of 15 ml/min/1.73 m$^2$ or more and less than 45 ml/min/1.73 m$^2$.

4. The drug according to any one of claims 1 to 3, wherein the primary glomerular disease or nephrosclerosis patient has a plasma concentration of 50 pg/ml or more on average 2 to 6 hours after administration of the sustained-release preparation comprising the compound represented by the general formula (I) as an active ingredient once after a meal at 120 μg as the compound represented by the general formula (I).

5. The drug according to any one of claims 1 to 4, which is combined with an angiotensin-converting enzyme inhibitor as an active ingredient.

6. The drug according to any one of claims 1 to 4, which is used to be administered simultaneously, separately, or sequentially with a different preparation comprising an angiotensin-converting enzyme inhibitor as an active ingredient.

7. The drug according to any one of claims 1 to 4, which is combined preparations that are used to be administered simultaneously, separately, or sequentially in treatment or prophylaxis for preventing dialysis shift or renal death, the drug separately comprising the following two preparations (a) and (b):

   (a) an oral sustained-release preparation comprising the compound represented by the general formula (I) as an active ingredient; and
   (b) a preparation comprising an angiotensin-converting enzyme inhibitor as an active ingredient.

8. The drug according to any one of claims 1 to 4, which is used in combination with an angiotensin-converting enzyme inhibitor.

9. A drug for preventing dialysis shift or renal death, which is a sustained-release preparation comprising, as an active ingredient, a compound represented by the following general formula (I):

[Formula 2]

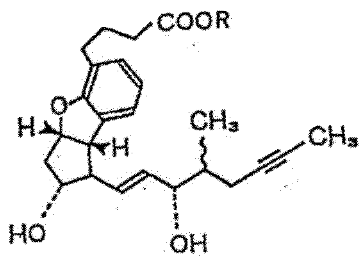

... (I)

wherein R represents hydrogen or a pharmacologically acceptable cation,
wherein the drug is used in such a manner that the compound represented by the general formula (I) is administered at 220 to 260 μg per day to a primary glomerular disease or nephrosclerosis patient with a nutritional disorder.

10. The drug according to claim 9, wherein the nutritional disorder is protein energy wasting (PEW) or a preliminary disease thereof.

11. The drug according to claim 9 or 10, wherein the nutritional disorder satisfies at least one of four constituent elements of PEW.

12. The drug according to any one of claims 9 to 11, wherein the nutritional disorder is cachexia, sarcopenia, or frailty.

Fig. 1

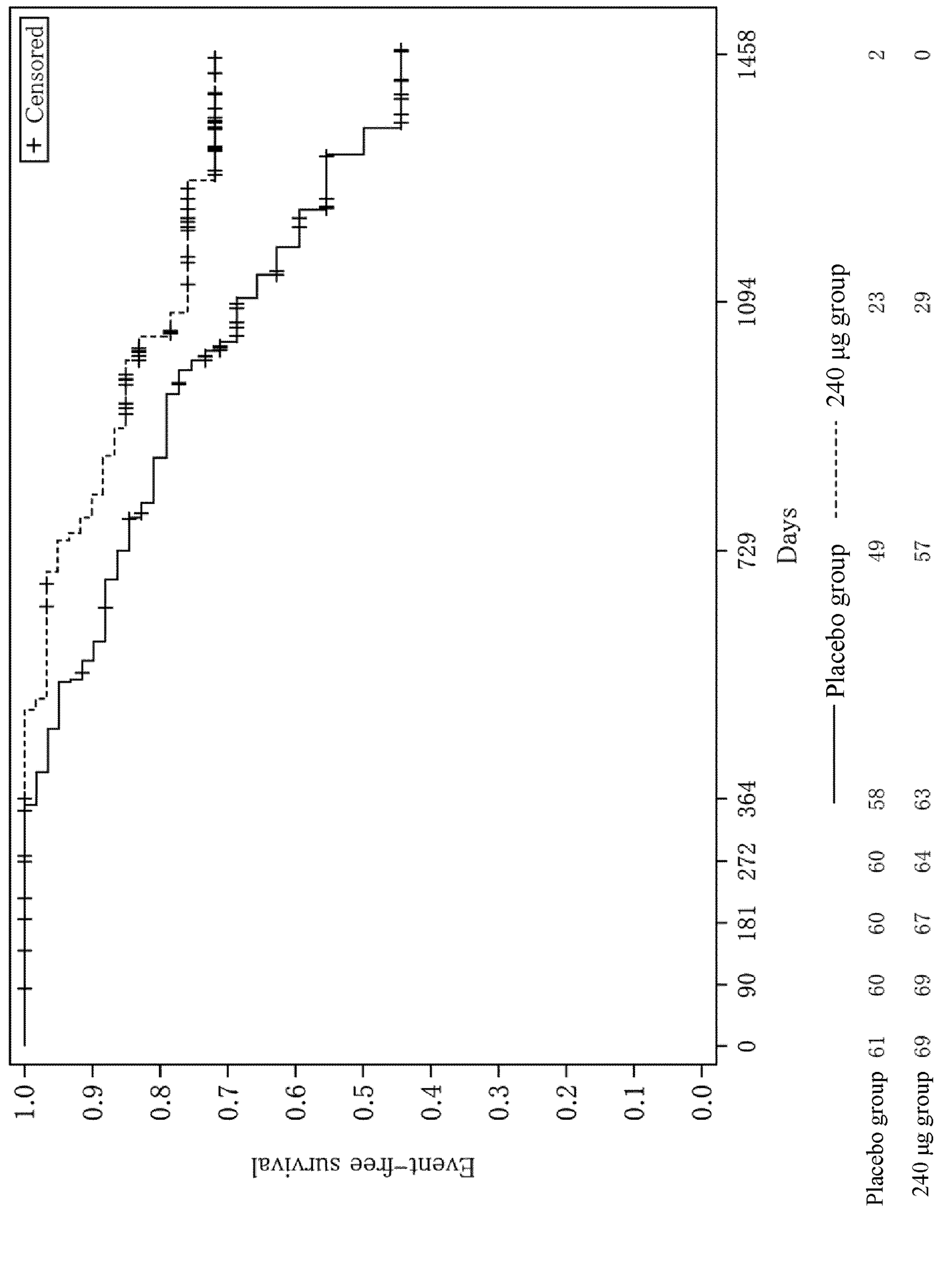

Event-free survival
1.0
0.9
0.8
0.7
0.6
0.5
0.4
0.3
0.2
0.1
0.0
+ Censored
Days
0
90
181
272
364
729
1094
1458
Placebo group
240 µg group
Placebo group 61 60 60 60 58 49 23 2
240 µg group 69 69 67 64 63 57 29 0

Fig. 2

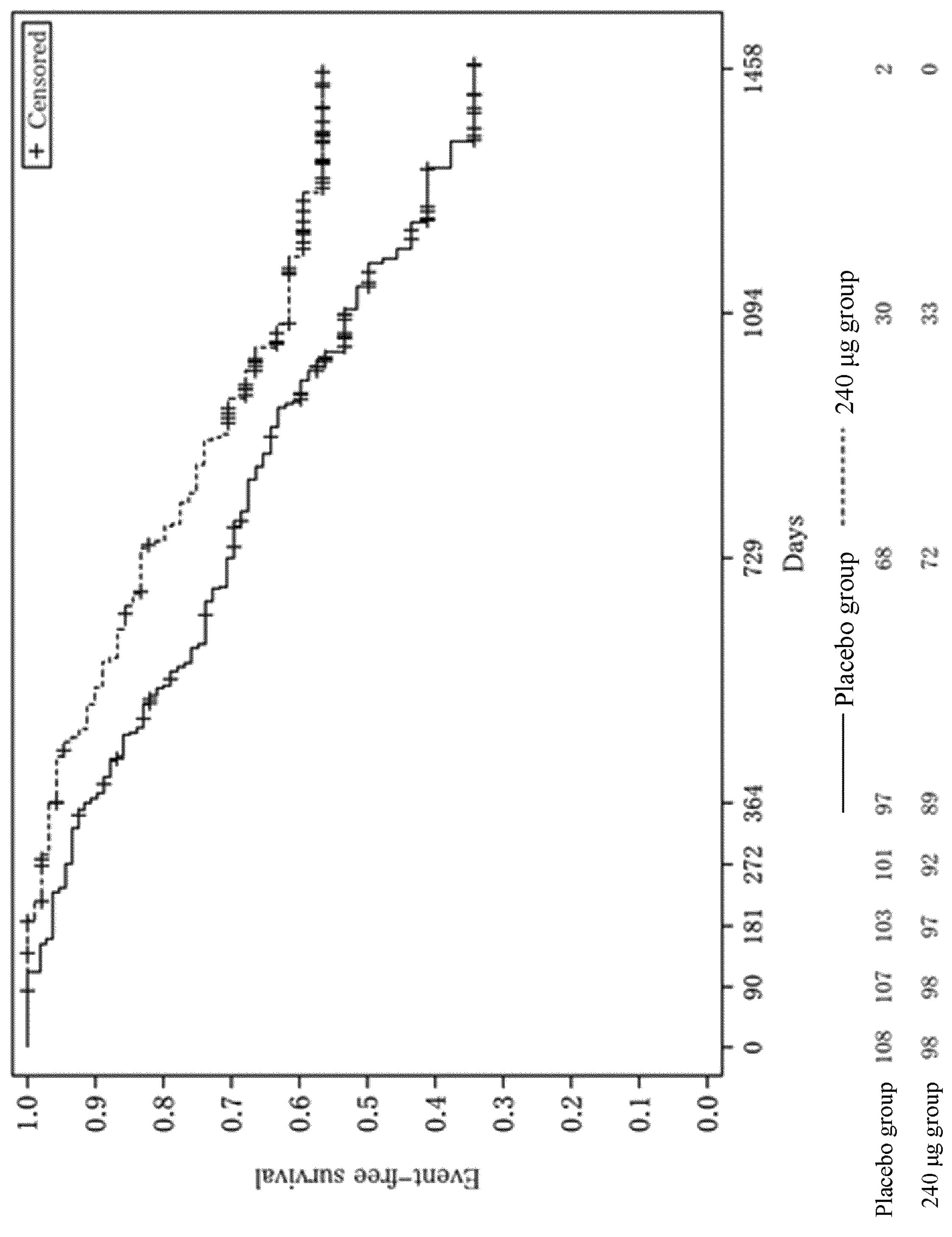
+ Censored
Event-free survival
1.0
0.9
0.8
0.7
0.6
0.5
0.4
0.3
0.2
0.1
0.0
0
90
181
272
364
729
1094
1458
Days
Placebo group
240 µg group
Placebo group 108 107 103 101 97 68 30 2
240 µg group 98 98 97 92 89 72 33 0

Fig. 3

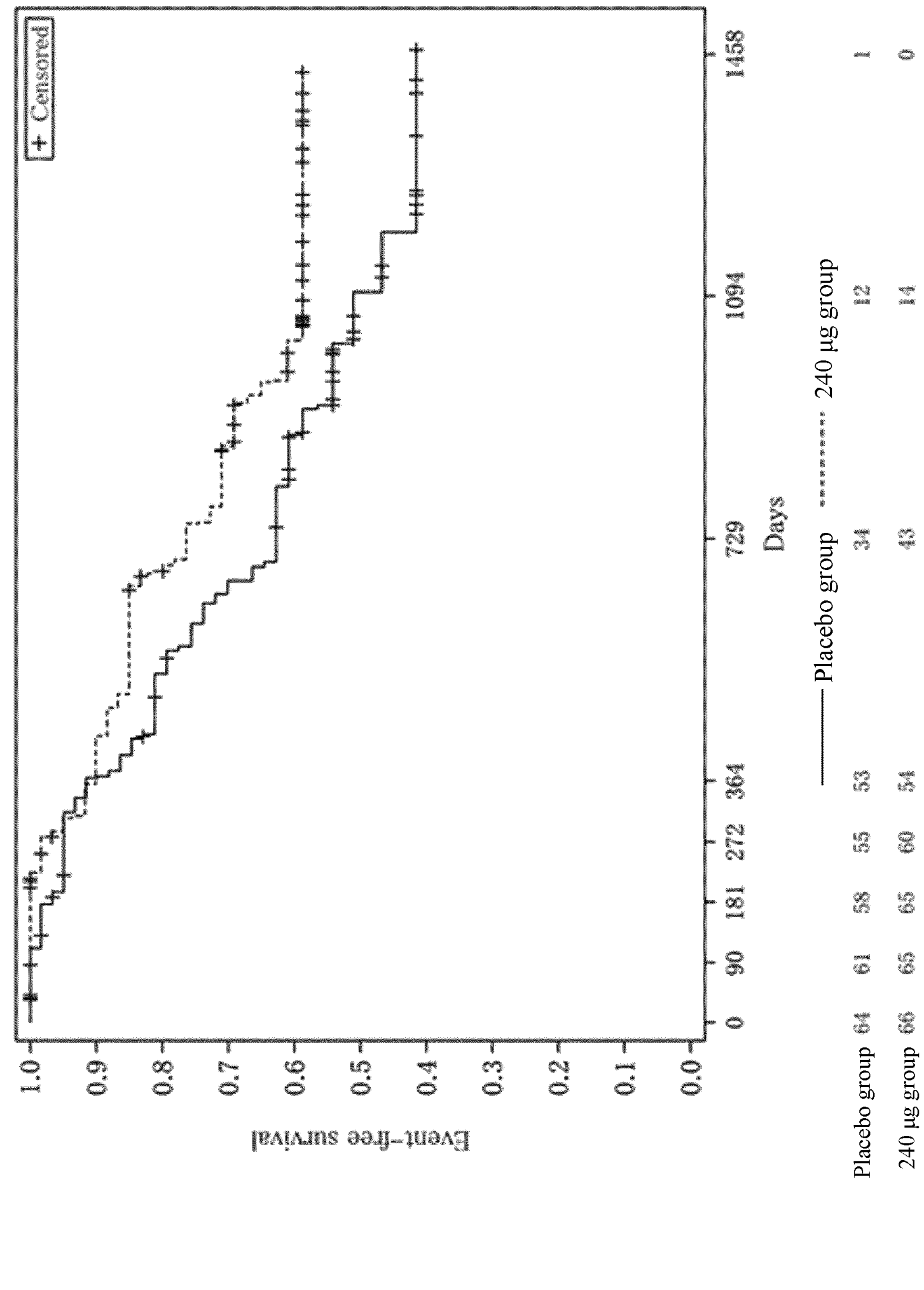
Event-free survival
1.0
0.9
0.8
0.7
0.6
0.5
0.4
0.3
0.2
0.1
0.0
+ Censored
0
90
181
272
364
729
1094
1458
Days
Placebo group
240 µg group
Placebo group 64 61 58 55 53 34 12 1
240 µg group 66 65 65 60 54 43 14 0

Fig. 4

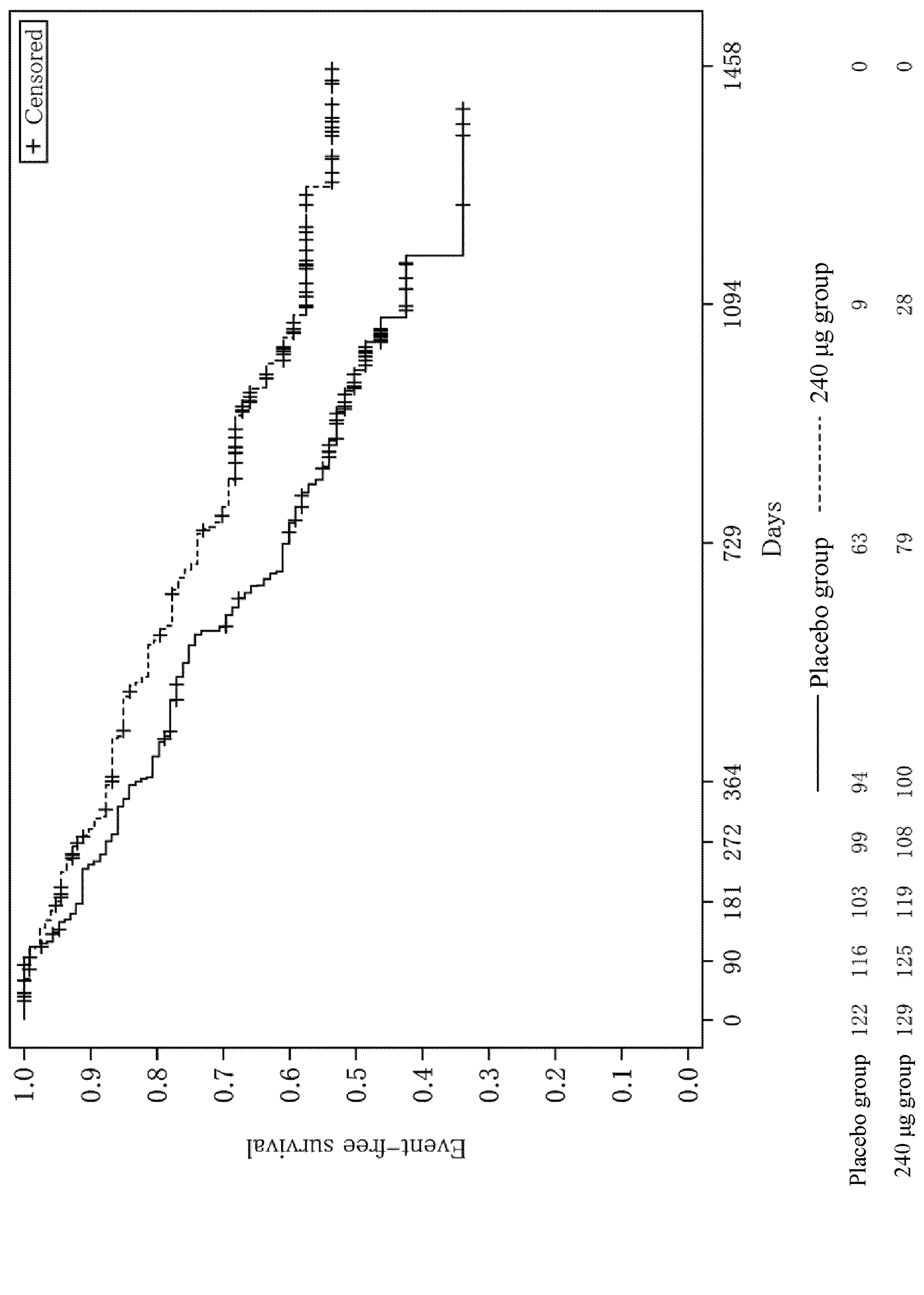

+ Censored
Event−free survival
1.0
0.9
0.8
0.7
0.6
0.5
0.4
0.3
0.2
0.1
0.0
0
90
181
272
364
729
1094
1458
Days
Placebo group
240 µg group
Placebo group 122 116 103 99 94 63 9 0
240 µg group 129 125 119 108 100 79 28 0

Fig. 5

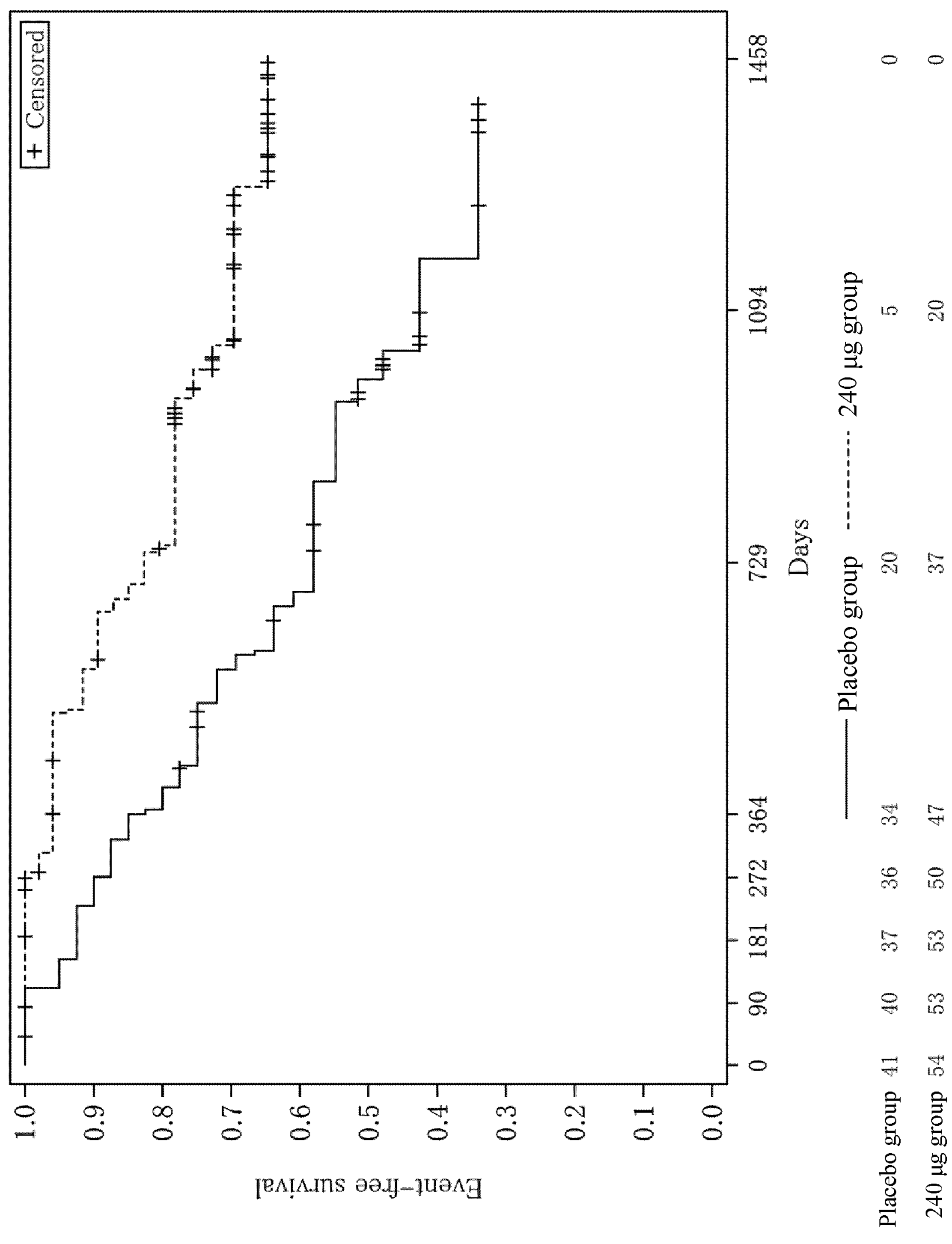
+ Censored
Event−free survival
1.0
0.9
0.8
0.7
0.6
0.5
0.4
0.3
0.2
0.1
0.0
0
90
181
272
364
729
1094
1458
Days
Placebo group
240 µg group
Placebo group 41 40 37 36 34 20 5 0
240 µg group 54 53 53 50 47 37 20 0

Fig. 6

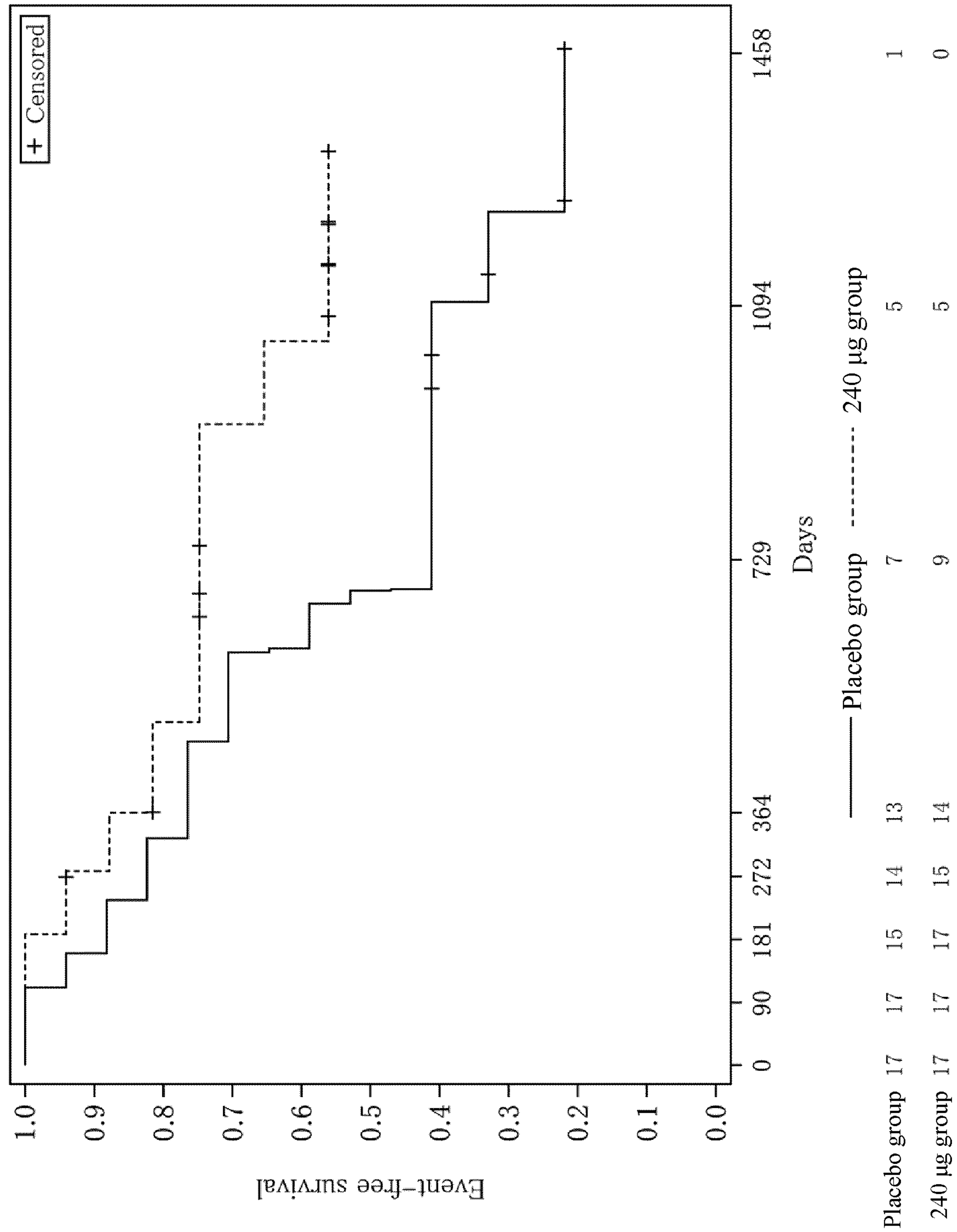

+ Censored
Event−free survival
1.0
0.9
0.8
0.7
0.6
0.5
0.4
0.3
0.2
0.1
0.0
0
90
181
272
364
729
1094
1458
Days
Placebo group
240 µg group
Placebo group 17 17 15 14 13 7 5 1
240 µg group 17 17 17 15 14 9 5 0

Fig. 7

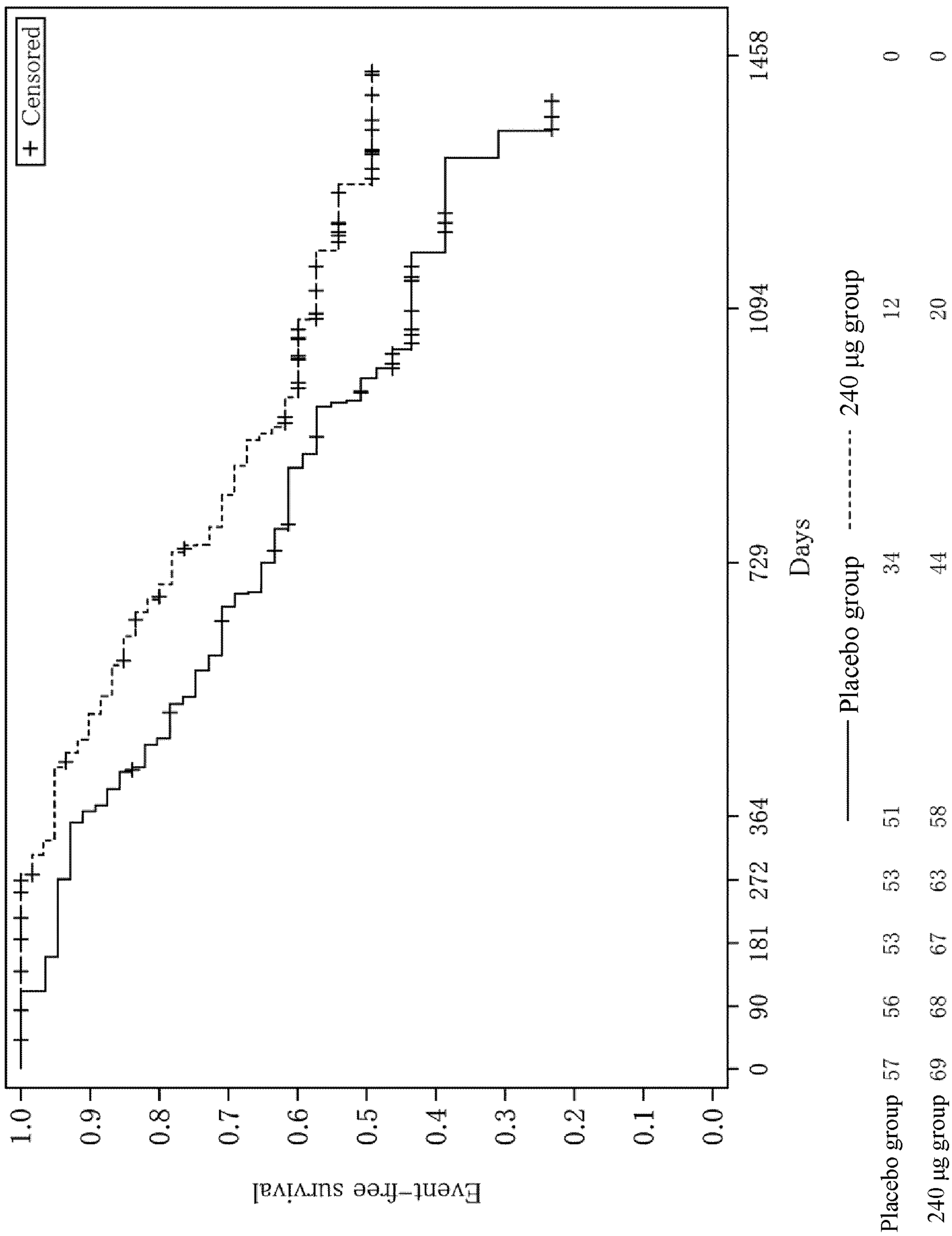

+ Censored
Event-free survival
1.0
0.9
0.8
0.7
0.6
0.5
0.4
0.3
0.2
0.1
0.0
0
90
181
272
364
729
1094
1458
Days
Placebo group
240 µg group
Placebo group 57 56 53 53 51 34 12 0
240 µg group 69 68 67 63 58 44 20 0

**INTERNATIONAL SEARCH REPORT**

International application No.
PCT/JP2020/048091

A. CLASSIFICATION OF SUBJECT MATTER
A61K 31/5585(2006.01)i; A61K 9/06(2006.01)i; A61K 9/20(2006.01)i; A61K 45/00(2006.01)i; A61K 47/38(2006.01)i; A61P 13/12(2006.01)i; A61P 43/00(2006.01)i
FI: A61K31/5585; A61P13/12; A61K45/00; A61P43/00 121; A61P43/00 111; A61K9/06; A61K47/38; A61K9/20
According to International Patent Classification (IPC) or to both national classification and IPC

B. FIELDS SEARCHED
Minimum documentation searched (classification system followed by classification symbols)
A61K31/5585; A61K9/06; A61K9/20; A61K45/00; A61K47/38; A61P13/12; A61P43/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII);
CAplus/REGISTRY/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| A | KOYAMA, Akio et al., "Orally active prostacyclin analogue beraprost sodium in patients with chronic kidney disease: a randomized, double-blind, placebo-controlled, phase II dose finding trial", BMC Nephrology, 2015, 16: 165, DOI:10.1186/s12882-015-0130-5 entire text, all drawings | 1-12 |
| A | NAKAMOTO, Hidemoto et al., "A multinational phase IIb/III trial of beraprost sodium, an orally active prostacyclin analogue, in patients with primary glomerular disease or nephrosclerosis (CASSIOPEIR trial), rationale and study design", BMC Nephrology, 2014, 15: 153, DOI: 10.1186/1471-2369-15-153 entire text, all drawings | 1-12 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 February 2021 (02.02.2021) | 16 February 2021 (16.02.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2020/048091

C (Continuation). DOCUMENTS CONSIDERED TO BE RELEVANT

| Category* | Citation of document, with indication, where appropriate, of the relevant passages | Relevant to claim No. |
|---|---|---|
| A | WO 2016/031949 A1 (TORAY INDUSTRIES, INC.) 03 March 2016 (2016-03-03) entire text, all drawings | 1-12 |
| A | JP 2005-232003 A (TORAY INDUSTRIES, INC.) 02 September 2005 (2005-09-02) entire text, all drawings | 1-12 |
| A | WO 2004/098611 A1 (TORAY INDUSTRIES, INC.) 18 November 2004 (2004-11-18) entire text, all drawings | 1-12 |
| A | WO 2000/67748 A1 (TORAY INDUSTRIES, INC.) 16 November 2000 (2000-11-16) entire text, all drawings | 1-12 |
| A | NAKAMOTO, Hidemoto et al., "Effects of Sustained-Release Beraprost in Patients With Primary Glomerular Disease or Nephrosclerosis: CASSIOPEIR Study Results", Therapeutic Apheresis and Dialysis, February 2020, 24 (1), pp. 42-55, DOI: 10.1111/1744-9987.12840 entire text, all drawings | 1-12 |

Form PCT/ISA/210 (continuation of second sheet) (January 2015)

**INTERNATIONAL SEARCH REPORT**
Information on patent family members

International application No.
PCT/JP2020/048091

| Patent Documents referred in the Report | Publication Date | Patent Family | Publication Date |
|---|---|---|---|
| WO 2016/031949 A1 | 03 Mar. 2016 | TW 20161:3606 A<br>entire text, all drawings | |
| JP 2005-232003 A | 02 Sep. 2005 | WO 2002/080929 A1<br>entire text, all drawings | |
| WO 2004/098611 A1 | 18 Nov. 2004 | US 2006/0217421 A1<br>entire text, all drawings<br>US 2009/0176848 A1<br>US 2017/0080089 A1<br>EP 1627638 A1<br>KR 10-2006-0003083 A<br>CN 1784235 A<br>CN 101439039 A | |
| WO 2000/67748 A1 | 16 Nov. 2000 | US 2006/0106095 A1<br>entire text, all drawings<br>US 2009/0163584 A1<br>EP 1106176 A1<br>CN 1317962 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2000067748 A
- WO 2004098611 A
- WO 9841210 A
- WO 2004103350 A


### Non-patent literature cited in the description


- **TAKENAKA et al.** *J Vet Intern Med,* 2018, vol. 32, 236
- **FUJITA et al.** *Prostaglandins, Leukotrienes and Essential Fatty Acids,* 2001, vol. 65 (4), 223-227
- **FUJITA et al.** *Vascular Biology & Medicine,* 2006, vol. 7, 281
- **KOYAMA et al.** *BMC Nephrology,* 2015, vol. 16, 165
- **NAKAMOTO et al.** *BMC Nephrology,* 2014, vol. 15, 153
- **NAKAMOTO et al.** *Ther Apher Dial.,* 23 May 2019
- **LEVEY et al.** *Am J Kidney Dis.,* 2014, vol. 64, 821-835
- **HAMADA et al.** *Shikoku Acta Medica,* 2013, vol. 69 (5,6), 211-214
- *Clinical Guidelines for Sarcopenia,* 2017, 2
- Study on Health Business for Late-Stage Elderly. *2015 Summary/Shared Research Report,* 2015
- **NOFUJI et al.** *Community Medicine,* 2018, vol. 32 (4), 312-320
- **KAJIKAWA et al.** *Arzneimittelforschung,* 1989, vol. 39, 495-9
- **SHIMAMURA et al.** *J Clin Pharmacol.,* 2017, vol. 57, 524-535
- **LEVEY et al.** *Ann Intern Med,* 2009, vol. 150, 604-612
- Evidence-based Practice Guideline for the Treatment of CKD. Japanese Society of Nephrology, 2009, 3
- Pharmaceutical Glossary. Pharmaceutical Society of Japan